# EUROPEAN PATENT APPLICATION

(11) **EP 0 711 784 A1**
(43) Date of publication of application: **15.05.1996**
(21) Application number: 94500175.8
(22) Date of filing: 08.11.1994
(51) Int. Cl.: C07H 15/24, C12P 19/44, C12R 1/645, A61K 31/705

(54) **Antifungal Sordarin derivatives**

(71) Applicant: GLAXO, S.A., E-28760 Tres Cantos, Madrid (ES)
(72) Inventor: Hayes, Michael, Greenford, Middlesex UB6 0HE (GB); Wildman, Howard, Greenford, Middlesex UB6 0HE (GB); Dawson, Michael, Greenford, Middlesex UB6 0HE (GB); Hall, Richard, Greenford, Middlesex UB6 0HE (GB); Garcia-Ochoa Dorado, Silvestre, E-28760 Tres Cantos, Madrid (ES); Bueno Calderon, José Maria, E-28760 Tres Cantos, Madrid (ES)
(74) Representative: Carpintero Lopez, Francisco

(57) **Abstract**

Sordarin derivatives of formula (I): 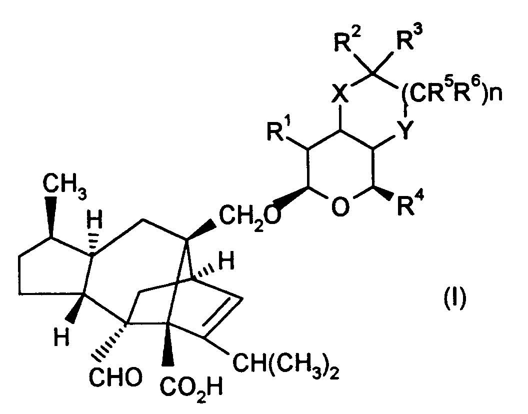
have antifungal activity

## Description

This invention relates to novel sordarin derivatives having antifungal activity, to processes for their preparation, to pharmaceutical compositions containing them and to their use in medicine, more particularly in the prevention or treatment of diseases in animals, including humans, caused by fungal infection.

British Patent Specification No. 1,162,027 describes the preparation of an antibiotic, SL2266, by the cultivation of the strain NRRL 3196 of the fungus species Sordaria araneosa. SL 2266, later named sordarin, is reported to have fungistatic activity. The same research group also described in Helvetica Chimica Acta (1971), 51, 119-120 the degradation of sordarin to sordaricin. Published Japanese Patent Application No. J6 2040292A describes the preparation of an antibiotic, zofimarin, which is reported to have antifungal activity.

Sordarin, sordaricin and zofimarin may be represented by formula (A) below 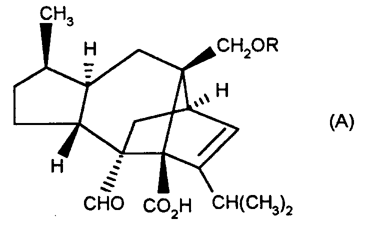
where
OR as 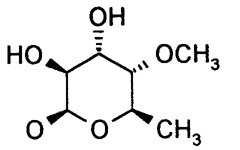
describes sordarin;
OR as OH describes sordaricin; and
OR as 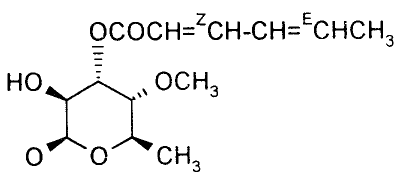
describes zofimarin.

Although sordarin and zofimarin exhibit antifungal activity, both compounds are only moderately active and have limited spectra of action when tested against a battery of fungal organisms. We now describe hereinafter a novel group of fungicidal sordarin derivatives which exhibit excellent antifungal activity and a broad spectrum of action. Thus, according to a first aspect of the present invention, we provide compounds of formula (I) 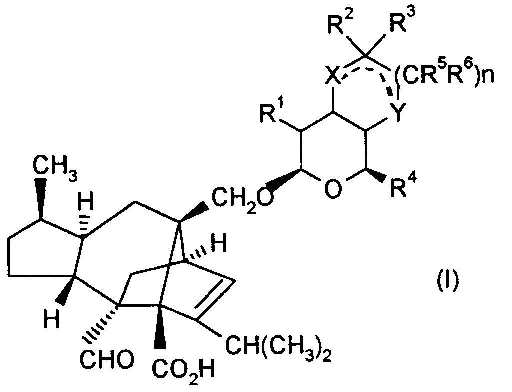
and pharmaceutically acceptable salts and solvates (e.g. hydrates) thereof, wherein R¹ represents hydrogen, halogen, hydroxyl, C₁₋₄alkoxy or acyloxy;
R² and R³ may each independently represent hydrogen, C₁₋₆alkyl or C₁₋₄alkoxyC₁₋₄alkyl or R² and R³ may together with the carbon atom to which they are attached represent C=O, C=S or C₃₋₈cycloalkyl;
R⁴ represents hydrogen or CH₂R⁷ (where R⁷ is hydrogen, hydroxyl, C₁₋₄alkoxy or a group OCOR⁸ in which R⁸ is C₁₋₄alkyl or aryl);
R⁵ and R⁶ may each independently represent hydrogen, C₁₋₆alkyl or C₁₋₄alkoxyC₁₋₄alkyl or R⁵ and R⁶ may together with the carbon atom to which they are attached represent C=O, C=S or C₃₋₈cycloalkyl;
n represents zero or 1;
X and Y may each independently represent oxygen, sulphur or CR⁹R¹⁰ (where R⁹ and R¹⁰ may each independently represent hydrogen, C₁₋₆alkyl or C₁₋₄alkoxyC₁₋₄alkyl or R⁹ and R¹⁰ may together with the carbon atom to which they are attached represent C=O, C=S, C₃₋₈cycloalkyl or C=CHR¹¹ where R¹¹ represents hydrogen or C₁₋₄alkyl); or when X or Y is oxygen and n is zero then -Y-CR²R³ or -X―CR²R³- respectively may also represent -N=CR³- or -NR¹²-CR²R³- (where R² and R³ are as defined previously and R¹² is C₁₋₄alkyl or an acyl group COR¹³ where R¹³ is C₁₋₆alkyl); and the dotted line in formula (I) indicates the optional presence of an additional bond.

Suitable pharmaceutically acceptable salts of the compounds of formula (I) include inorganic base salts such as alkali metal salts (for example sodium and potassium salts) and ammonium salts and organic base salts. Suitable organic base salts include amine salts such as trialkylamine (e.g. triethylamine), dialkylamine (e.g. dicyclohexylamine), optionally substituted benzylamine (e.g. phenylbenzylamine or p-bromobenzylamine), procaine, ethanolamine, diethanolamine, N-methylglucosamine and tri(hydroxymethyl)methylamine salts and amino acid salts (e.g. lysine and arginine salts).

References hereinafter to a compound of formula (I) includes that compound and its pharmaceutically acceptable salts.

Other salts which are not pharmaceutically acceptable may be useful in the preparation of compounds of formula (I) and these form a further aspect of the invention.

It is to be understood that the present invention encompasses any individual isomers, including optical isomers, of compounds represented by formula (I) above as well as mixtures thereof, including wholly or partially racemic mixtures thereof.

As used herein, "alkyl" as a group or part of a C₁₋₄alkoxy group may be a straight or branched chain comprising, for example, 1 to 4 carbon atoms. Suitable examples include methyl, ethyl, n-propyl, i-propyl, n-butyl, s-butyl and t-butyl.

As used herein, the term "aryl" as a group or part of a group means phenyl or heteroaryl each optionally substituted by one or more (e.g. 1, 2 or 3) atoms or groups selected from halogen, hydroxyl, C₁₋₆alkyl, C₁₋₆alkoxy or C₁₋₄alkoxycarbonyl. The heteroaryl group may be a 5- or 6-membered heteroaromatic ring containing one or more heteroatoms selected from nitrogen, oxygen and sulphur. Suitable examples of heteroaryl groups include pyridyl, fury, thienyl and pyrrolyl.

The term "halogen" means herein fluorine, chlorine, bromine or iodine.

When R¹ is an acyloxy group it may represent, for example, a group OCOR¹³ where R¹³ is as defined above.

Examples of C₃₋₈cycloalkyl groups include cyclopentyl and cyclohexyl groups.

R¹ may represent, for example, a hydrogen atom or a hydroxyl group.

R² may represent, for example, hydrogen or C₁₋₄alkyl (e.g. methyl), and R³ may represent, for example, hydrogen, C₁₋₄alkyl (e.g. methyl, ethyl or n-propyl) or C₁₋₄alkoxyC₁₋₄alkyl (e.g. methoxyethyl), or CR²R³ may represent C=O, C=S or C₃₋₈cycloalkyl (e.g. cyclopentyl).

R⁴ may represent, for example, methyl or C₁₋₄alkoxymethyl (e.g. methoxymethyl).

R⁵ and R⁶ may each independently represent, for example, hydrogen or C₁₋₄alkyl (e.g. methyl).

Examples of ring systems represented by 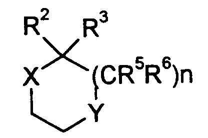
include

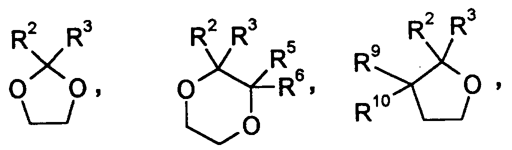

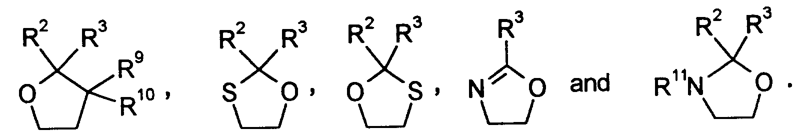

A particular group of compounds of the invention are compounds of formula (Ia) 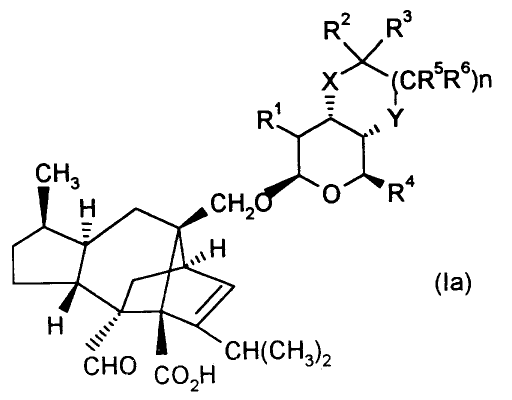
and pharmaceutically acceptable salts and solvates (e.g. hydrates) thereof, wherein R¹ to R⁶, n, X and Y are as defined in formula (I) above.

When R¹ represents hydroxyl or C₁₋₄alkoxy, the R¹ moiety is preferably sited in the axial configuration. However, R¹ preferably represents a hydrogen atom.

R² and R³ may each particularly represent individually hydrogen or C₁₋₄alkyl.

R⁴ preferably represents methyl.

n preferably represents zero.

Particular ring systems represented by 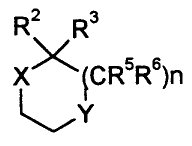
include 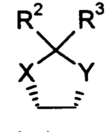
where R² and R³ are as defined previously and X and Y are each independently oxygen, sulphur or CR⁹R¹⁰ (where R⁹ and R¹⁰ are as defined previously), provided that at least one of X and Y is oxygen.

Preferred ring systems include 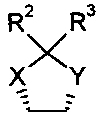
where R² and R³ are as defined previously and one of X and Y represents oxygen and the other represents CR⁹R¹⁰ (where R⁹ and R¹⁰ each independently represent hydrogen or C₁₋₄alkyl (e.g. methyl) or X and Y both represent oxygen.

It is to be understood that the present invention covers all combinations of particular and preferred groups described hereinabove.

A further particular group of compounds of the invention are compounds of formula (Ib) 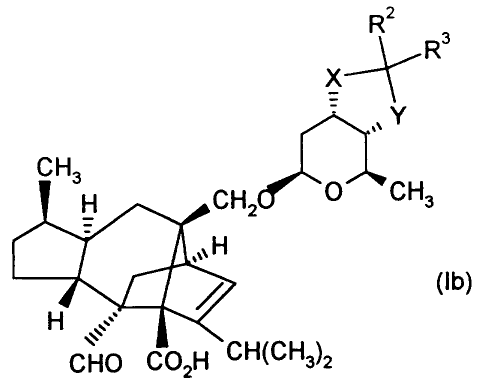
and pharmaceutically acceptable salts and solvates (e.g. hydrates) thereof, wherein R² and R³ are as defined previously and one of X and Y represents oxygen and the other represents CR⁹R¹⁰ [where R⁹ and R¹⁰ each independently represent hydrogen or C₁₋₄alkyl (e.g. methyl)] or X and Y both represent oxygen. Within formula (Ib) above, when X and Y both represent oxygen then R² and R³ both preferably represent C₁₋₄alkyl (e.g. methyl), and when one of X and Y represents oxygen and the other represents CR⁹R¹⁰ then R² and R³ preferably each independently represent hydrogen or C₁₋₄alkyl.

Specific compounds according to the present invention include:
[1R-(1α,3aβ,4β,4aβ,7β,7aα,8aβ)] 8a-[(2,6-dideoxy-3,4-O-isopropylidene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid;
[1R-(1α,3aβ,4β,4aβ,7β,7aα,8aβ)] 8a-[[1S,4S,6R,8R]-2,7-dioxa-4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid; and
[1R-(1α,3aβ,4β,4aβ,7β,7aα,8aβ)] 8a-[[1S,4R,7R,9R]-2,8-dioxa-4,9-dimethyl-cis-bicyclo[3.4.0]-non-7-yl-oxy-methyl]-4-formyl-4,4a,5,6,7,7a,8,8a-octahydro-7-methyl-3-(1-methylethyl)-1,4-methano-s-indacene-3a(1H)-carboxylic acid; and
pharmaceutically acceptable salts and solvates (e.g. hydrates) thereof.

The compounds of formula (I) are very active fungicides useful in combatting fungal infections in animals, including humans. For example, they may be used in the treatment of systemic infections caused by organisms such as species of Candida (e.g. Candida albicans, Candida glabrata, (Torulopsis glabrata), Candida tropicalis, Candida parapsilosis and Candida pseudotropicalis), Cryptococcus neoformans, Pneumocystis carinii, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be used to treat topical infections caused by species of Candida, Trichophyton, Microsporum or Epidermophyton (e.g. Trichophyton mentographytes, Trichophyton rubrum, Microsporum canis or Epidermophyton floccosum), or in mucosal infections caused by Candida albicans.

Compounds of formula (I) may also be used to treat other infections caused by species of filamentous fungi such as Geotrichum (e.g. Geotrichum clavatum), Trichosporon (e.g. Trichosporon beigelii), Blastoschizomyces (e.g. Blastoschizomyces capitatus), Sporothrix (e.g. Sporothrix schenckii), Scedosporium (e.g. Scedosporium apiospetum), Cladosporium (e.g. Cladosporium carrionii) and Pityrosporum ovale.

The in vitro evaluation of the anti-fungal activity of compounds of the invention was performed on liquid or solid medium by the anti-fungal two-fold serial dilution technique of determining the minimum inhibitory concentration (MIC) of anti-fungal agent that inhibited development of growth after 24 to 48 hours of incubation at 37°C. In practice, a series of agar plates or broth microdilution panels containing two-fold dilutions of antifungal agent tested were inoculated with a standard culture of a clinically relevant pathogen, for example, candida albicans. The agar plates or broth microdulution panels were then examined for the presence or absence of growth of the fungus and the appropriate MIC values were noted.

MFC values (defined as the lowest anti-fungal concentration that killed at least 99.9% of the initial inoculum in liquid medium) may also be determined by sub-culturing 0.01 and 0.1µl of broth from the drug-free control well, the first well containing growth and each clear well on agar plates.

The in vivo evaluation of compounds of formula (I) can be carried out at a series of dose levels by administration (e.g. subcutaneously, orally, intraperitoneally or intravenously) to mice inoculated with a strain of Candida albicans. Untreated mice die within 3 to 9 days and the dose level at which the test compound provides 50% protection against the lethal effect of the infection is noted.

In view of their antifungal activity, compounds of formula (I) recommend themselves for the treatment of a variety of fungal infections in human beings and animals. Such infections include superficial, cutaneous, subcutaneous and systemic mycotic infections such as respiratory tract infections, gastrointestinal tract infections, cardiovascular infections, urinary tract infections, CNS infections, candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be used as prophylactic agents to prevent systemic and topical fungal infections. Use as prophylactic agents may, for example, be appropriate as part of a selective gut decontamination regimen in the prevention of infection in immunocompromised patients (e.g. AIDS patients, patients receiving cancer therapy or transplant patients). Prevention of fungal overgrowth during antibiotic treatment may also be desirable in some disease syndromes or iatrogenic states.

While it is possible that, for use in therapy, compounds of the invention may be administered as the raw chemical, it is preferable to present the active ingredient as a pharmaceutical formulation. The invention thus further provides a pharmaceutical formulation comprising compounds of formula (I) and physiologically acceptable salts thereof together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant, e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropriate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg, advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention may also be used in combination with other therapeutic agents, and the invention thus provides, in a further aspect, a combination comprising a compound of the invention together with another therapeutically active agent.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of formula (I) or a physiologically acceptable salt thereof or a pharmaceutical composition comprising a compound of formula (I) or a physiologically acceptable salt thereof as defined above for use in therapy, particularly for the treatment of fungal infections in animals (especially humans).

According to another aspect of the present invention, we provide the use of a compound of formula (I) or a physiologically acceptable salt thereof in the manufacture of a medicament for the treatment of fungal infections in a human or non-human animal patient.

According to a further aspect of the present invention, we provide a method of treatment of the human or non-human animal body to combat fungal diseases, which method comprises administering to said body an effective amount of a compound of formula (I) or a physiologically acceptable salt thereof.

It will be appreciated by those skilled in the art that references herein to treatment extend to prophylaxis as well as the treatment of established conditions or infections.

The compounds of the invention may be prepared by the processes described below.

Thus, a general process (A) for the preparation of a compound of formula (I) comprises reacting a compound of formula (II) 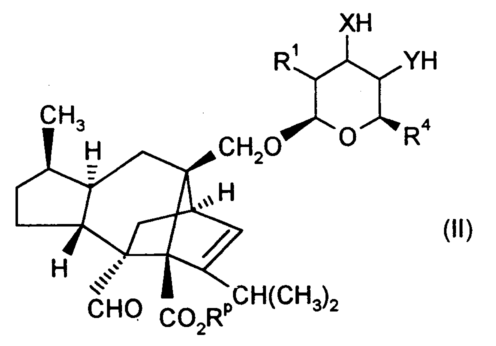
(in which R¹ and R⁴ are as defined in formula (I) above, R^{p} is a carboxyl protecting group and X and Y are as defined in formula (I) above, except that X and/or Y cannot represent CR⁹R¹⁰) to form the desired cyclic system, followed by the removal of the carboxyl protecting group.

According to a first embodiment of process (A), a compound of formula (Ia) in which R¹ to R⁴ are as defined in formula (I) above, n is zero and X and Y are both oxygen may be prepared by treating a diol of formula (III) 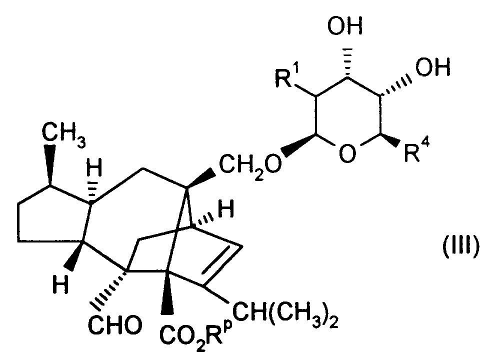
(in which R¹ and R⁴ are as defined in formula (I) above and R^{p} is a carboxyl protecting group) with a compound (L)₂CR²R³ (in which L is a suitable leaving group), followed by the removal of the carboxyl protecting group. When R² and R³ both represent hydrogen the ring-forming reaction may conveniently be effected by treating a compound of formula (III) with a dihalomethane (e.g. dibromomethane) in the presence of a strong base, such as an alkali metal hydroxide (e.g. sodium hydroxide), preferably under phase transfer conditions, using for example a tetraalkylammonium salt (e.g. tetrabutylammonium bromide), at about ambient temperature. When at least one of R² and R³ is a C₁₋₆alkyl group or a C₁₋₄alkoxyC₁₋₄alkyl group or CR²R³ is a C₃₋₈cycloalkyl group the ring-forming reaction may conveniently be effected by treating a compound of formula (III) with a ketal (RO)₂CR²R³ (wherein R is a C₁₋₆alkyl group, e.g. methyl), preferably in the presence of a suitable acid such as p-toluenesulphonic acid or pyridinium p-toluenesulphonate, and in a suitable solvent such as a ketone (e.g. acetone), a nitrile (e.g. acetonitrile) or a halogenated hydrocarbon (e.g. dichloromethane) at about room temperature. Compounds of formula (Ia) in which X and/or Y represents sulphur may be similarly prepared from intermediates corresponding to those of formula (III) in which one or both of the diol hydroxyl groups is replaced by thiol. When CR²R³ represents C=O or C=S the ring-forming reaction may conveniently be effected by reacting a compound of formula (III) with carbonyldiimidazole or thiocarbonyldiimidazole in a suitable solvent such as a hydrocarbon (e.g. toluene) or an ether (e.g. tetrahydrofuran) under reflux. Alternatively, when CR²R³ represents C=S the ring-forming reaction may be effected by treating a compound of formula (III) with a tin oxide (e.g. dibutyltin oxide) in a hydrocarbon solvent (e.g. refluxing toluene), followed by the addition of a halothionoformate (e.g. phenyl chlorothionoformate) in a hydrocarbon solvent (e.g. toluene) at about room temperature.

According to a further embodiment of process (A), a compound of formula (Ia) in which R¹ and R⁴ are as defined in formula (I) above, one of R² and R³ represents C₁₋₆alkyl and the other represents hydrogen, C₁₋₆alkyl or C₁₋₄alkoxyC₁₋₄alkyl, (CR⁵R⁶)n represents CR⁵R⁶ where R⁵ and R⁶ are as defined in formula (I) above and X and Y both represent oxygen may be prepared by treating a diol of formula (III) with a tin oxide (e.g. dibutyltin oxide) in a hydrocarbon solvent (e.g. refluxing toluene), followed by addition of an allylhalide HalCR⁵R⁶CR²=CHR¹⁴ (where R¹⁴ is hydrogen or C₁₋₆alkyl and Hal is halogen, e.g. bromine) and a fluoride salt (e.g. tetrabutylammonium fluoride) in a suitable solvent such as an ether (e.g. tetrahydrofuran), and heating the mixture at a temperature in the range of about 40° to 80°C to give a compound of formula (IV) 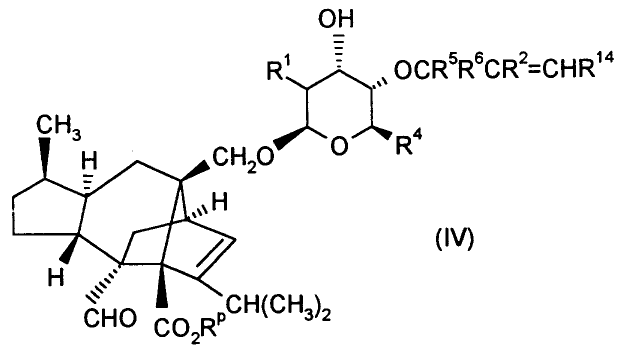
which may be converted to the desired compound of formula (Ia) by cyclisation involving an intramolecular electrophilic addition induced by a mercury salt (e.g. mercuric trifluoroacetate) followed by a hydride reduction, for example using a trialkyltin hydride (e.g. tributyltin hydride), and thereafter removing the carboxyl protecting group. The reaction may conveniently be effected at about room temperature in the presence of a suitable solvent such as an ether (e.g. tetrahydrofuran).

In another embodiment of process (A), a compound of formula (Ia) in which R¹ to R⁴ are as defined in formula (I) above, n is zero and one of X and Y represents oxygen and the other is CR⁹R¹⁰ (where one of R⁹ and R¹⁰ represents C₁₋₆alkyl and the other represents hydrogen, C₁₋₆alkyl or C₁₋₄alkoxyC₁₋₄alkyl or CR⁹R¹⁰ represents C=CHR¹¹) may be prepared by treating a compound of formula (III) with a tin oxide (e.g. dibutyltin oxide) in a hydrocarbon solvent (e.g. refluxing toluene) and optionally in the presence of a fluoride salt (e.g. tetrabutylammonium fluoride), and then either adding thereto an allylhalide HalCR²R³CR⁹=CHR¹⁴ or an alkynylhalide HalCR²R³C≡CR¹¹ (where R¹¹, R¹⁴ and Hal are as defined previously) or adding the allylhalide or alkynylhalide after protection of one of the diol hydroxyl groups, to give a compound of formula (V) 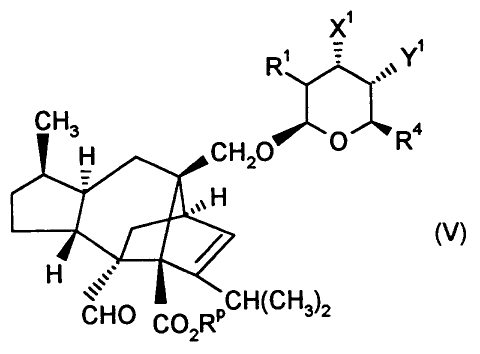
(where X¹ represents OH and Y¹ represents OCR²R³CR⁹=CHR¹⁴ or OCR²R³C≡CR¹¹, or X¹ represents OCR²R³CR⁹=CHR¹⁴ or OCR²R³C≡CR¹¹ and Y¹ represents a protected hydroxyl group). The cyclisation may be completed by first removing the hydroxyl protecting group, when present, then activating the free hydroxyl group, for example by forming an S-alkyldithiocarbonate (e.g. S-methyldithiocarbonate) and then closing the ring under radical conditions, for example by treating a solution of the activated intermediate in a hydrocarbon solvent (e.g. toluene) under reflux with a hydrogen donor, for example a hydride reducing agent (e.g. a trialkyltin hydride such as tributyltin hydride) in the presence of an activating agent [e.g. azobis(isobutyronitrile)], and thereafter removing the carboxyl protecting group. The formation of an S-alkyldithiocarbonate may conveniently be effected by treating a compound of formula (V) in which one of X¹ and Y¹ is OH and the other is OCR²R³CR⁹=CHR¹⁴ or OCR²R³C≡CR¹¹ with a strong alkali metal base (e.g. sodium hydride), in the presence of imidazole, and in a suitable solvent such as an ether (e.g. tetrahydrofuran) at a reduced temperature (e.g. about 0°C), and then adding carbon disulphide and an alkyl halide (e.g. methyl iodide) at about room temperature.

In a further embodiment of process (A), a compound of formula (Ia) in which R¹ and R⁴ are as defined in formula (I) above, R² and R³ each independently represent hydrogen, C₁₋₆alkyl or C₁₋₄alkoxyC₁₋₄alkyl or CR²R³ represents C₃₋₈cycloalkyl, n is zero, and one of X and Y represents -NR¹²-(where R¹² is as defined in formula (I) above) and the other represents oxygen may be prepared by N-acylation of a compound of formula (VI) 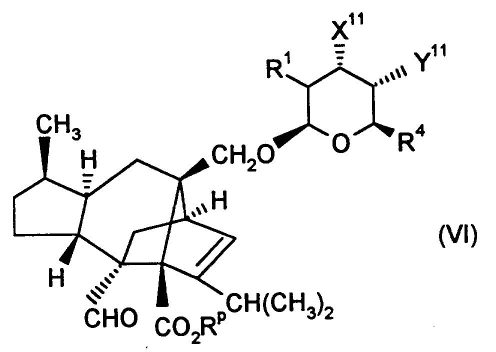
(in which one of X¹¹ and Y¹¹ is OH and the other is NH₂) or a protected derivative thereof, for example using an acylhalide such as an acyl chloride under conventional conditions, followed by treating the amide with an aldehyde or ketone R²R³C=O or a dialkylacetal R²R³C(OAlkyl)₂ in the presence of a suitable acid such as p-toluenesulphonic acid or pyridinium p-toluenesulphonate and in a suitable solvent such as a halogenated hydrocarbon (e.g. dichloromethane) at about room temperature, and thereafter removing any protecting groups present.

In another embodiment of process (A), a compound of formula (Ia) in which R¹ and R⁴ are as defined in formula (I) above, n is zero, -X―CR²R³ or -Y-CR²R³ represents -N=CR³- (where R³ is C₁₋₆alkyl or C₁₋₄alkoxyC₁₋₄alkyl), and Y or X remaining is oxygen may be prepared by treating a compound of formula (VI) with an imino ester R³C(=NH)OR (where R is a C₁₋₆alkyl group, e.g. methyl) in a halogenated hydrocarbon solvent (e.g. dichloromethane) at about 40°C, and thereafter removing any protecting groups present.

Another general process (B) comprises an interconversion reaction wherein a compound of formula (I) is prepared from a different compound of formula (I) or a protected derivative thereof.

According to a first embodiment of process (B), a carboxyl protected derivative of a compound of formula (I) in which R¹ is hydroxyl may be converted to a corresponding compound of formula (I) in which R¹ is hydrogen by a procedure comprising (i) forming an S-alkyldithiocarbonate according to the method described previously and (ii) removing this group by treating a solution of the intermediate compound in a hydrocarbon solvent (e.g. toluene) at an elevated temperature (e.g. about 80° to 120°C) with a hydride reducing agent (e.g. a trialkyltin hydride such as tributyltin hydride), and thereafter removing the carboxyl protecting group.

In a further embodiment of process (B), a compound of formula (I) in which R¹ is C₁₋₄alkoxy and/or R⁴ is C₁₋₄alkoxymethyl may be prepared by alkylating a protected derivative of a compound of formula (I) in which R¹ is hydroxyl and/or R⁴ is hydroxymethyl and any labile groups (e.g. carboxyl and hydroxyl groups) are protected, followed by removal of the protecting groups present. The alkylation may conveniently be effected by initial reaction with a strong alkali metal base (e.g. sodium hydride) and thereafter with an alkyl halide (e.g. methyl iodide). The reaction may be carried out in a suitable solvent such as an ether (e.g. tetrahydrofuran) at a temperature within the range of about 0° to 30°C.

In another embodiment of process (B), a compound of formula (I) in which R¹ is acyloxy and/or R⁴ is CH₂OCOR⁸ may be prepared by acylating a protected derivative of a compound of formula (I) in which R¹ is hydroxyl and/or R⁴ is CH₂OH and any labile groups (e.g. carboxyl and hydroxyl groups) are protected, followed by removal of any protecting groups present. The acylation reaction may be carried out using conventional methodology, for example by treatment with a carboxylic acid in the presence of an activating agent such as dicyclohexylcarbodiimide and a suitable base such as dimethylaminopyridine or using an acid halide (e.g. an acid chloride), optionally in the presence of a suitable base such as pyridine or 4-dimethylaminopyridine.

According to a further embodiment of process (B), a compound of formula (I) in which R¹ is a hydroxyl group in the equatorial configuration may be prepared from a protected derivative of a compound of formula (I) in which R¹ is a hydroxyl group in the axial configuration and any labile groups (e.g. carboxyl, hydroxyl and CHO groups) are protected, followed by removal of any protecting groups present. The isomerisation reaction may conveniently be effected by a two step procedure comprising (i) oxidising the 2'-axial OH to an oxo group by treatment with a suitable oxidising system [e.g. chromium oxide in the presence of pyridine in a solvent such as a halogenated hydrocarbon (e.g. dichloromethane) containing acetic anhydride] and (ii) reducing the oxo group to an equatorial OH group using a suitable reducing agent such as a borohydride (e.g. sodium borohydride). The reduction may conveniently be carried out in a suitable solvent such as an alcohol (e.g. aqueous methanol) at a temperature in the range of about 0° to 10°C.

In another embodiment of process (B), a compound of formula (I) in which R¹ is a halogen atom may be prepared from a protected derivative of a compound of formula (I) in which R¹ is a hydroxyl group, followed by removal of any protecting groups present. The displacement reaction takes place with inversion of configuration. Thus, for example, an axial OH group may conveniently be converted to an equatorial iodine atom by the addition of iodine to a solution of the starting material in a suitable solvent such as a hydrocarbon (e.g. toluene) in the presence of triphenyl phosphine and iodine, and then heating the mixture (e.g. under reflux).

A fluorine atom may similarly be introduced by treatment with a suitable fluorinating agent such as diethylaminosulphur trifluoride (DAST) in a suitable solvent such as a halogenated hydrocarbon (e.g. dichloromethane) or an aromatic hydrocarbon (e.g. toluene) at about room temperature.

In a yet further embodiment of process (B), a compound of formula (I) in which one of X and Y is oxygen and the other is sulphur, n is zero, R¹ and R⁴ are as defined in formula (I) above and CR²R³ is C=O may be prepared from a carboxyl protected derivative of a compound of formula (I) in which both X and Y are oxygen, n is zero, R¹ and R⁴ are as defined in formula (I) above and CR²R³ is C=S by treating said compound with a trialkyltin hydride (e.g. tributyltin hydride) in the presence of an activating agent [e.g. azobis(isobutyronitrile)], conveniently in a refluxing solvent such as a hydrocarbon (e.g. toluene), and thereafter removing the carboxyl protecting group.

Many of the above-mentioned procedures require the removal of one or more protecting groups as a final step to provide the desired compound of formula (I). Thus, a further general process (C) comprises deprotecting a protected derivative of a compound of formula (I). Suitable carboxyl protecting groups and hydroxyl protecting groups for use herein include any conventional protecting group, for example as described in "Protective Groups in Organic Chemistry", Ed. J. F. W. McOmie (Plenum Press, 1973) or "Protective Groups in Organic Synthesis" by Theodora W. Greene (John Wiley and Sons, 1991). Examples of suitable carboxyl protecting groups include aralkyl groups such as diphenylmethyl and silyl groups (e.g. trimethylsilylethyl). Examples of suitable hydroxyl protecting groups include arylalkyl groups such as p-methoxybenzyl and ester groups (e.g. benzyloxycarbonyl). Aldehyde groups may conveniently be protected in the form of cyclic ketals.

The protecting groups may be removed using conventional techniques. Thus, a diphenylmethyl group may conveniently be removed using trifluoroacetic acid or by hydrogenolysis in the presence of a palladium catalyst (e.g. 10% palladium on charcoal). A p-methoxybenzyl group may conveniently be removed using 2,3-dichloro-5,6-dicyano-1,4-benzoquinone. Silyl groups such as trimethylsilylethyl may conveniently be removed using fluoride ions. Cyclic ketal groups may conveniently be converted to aldehyde group by the addition of a suitable acid such as hydrochloric acid.

Compounds of formula (II) may conveniently be prepared by reacting a compound of formula (VII) 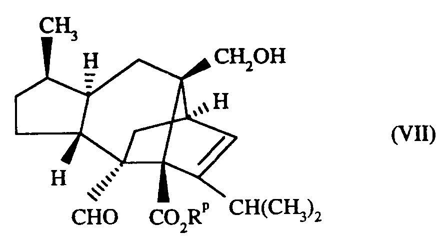
(where R^{p} is a carboxyl protecting group) with a compound of formula (VIII) 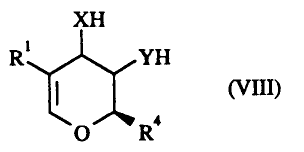
(where R¹, R⁴, X and Y are as defined in formula (II) above) or a protected derivative thereof, followed, where necessary, by removal of any protecting groups present. The reaction may conveniently be effected by heating (VII) and (VIII) at a temperature in the range of about 40° to 80°C in a suitable solvent, such as a hydrocarbon (e.g. toluene), in the presence of an acid such as hydrobromic acid-triphenylphosphine.

When XH and YH in formula (VIII) represent hydroxyl groups these may conveniently be protected as acyloxy (e.g. acetoxy) groups, with removal of the protecting groups following reaction with a compound of formula (VII). Removal of acetoxy protecting groups may conveniently be effected by addition of a suitable base such as an alkoxide (e.g. sodium methoxide) in a suitable solvent such as an alcohol (e.g. methanol) at about room temperature.

The aforementioned reaction is particularly suitable for preparing compounds of formula (II) in which R⁴ is other than a methyl group. When R⁴ is methyl it may be more convenient to prepare compounds of formula (II) from 4'-demethylsordarin, a compound of formula (IX) 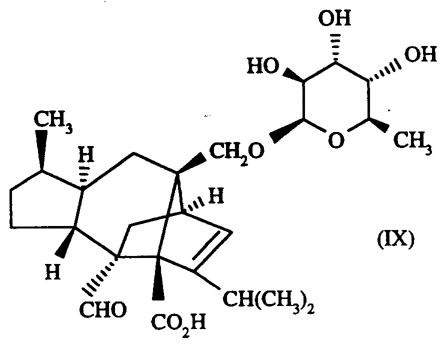
Thus, for example, compounds of formula (III) in which R⁴ represents methyl may be prepared by protection of the carboxyl group in (IX) using conventional methods, followed, if appropriate, by converting the 2'-axial hydroxyl group to a different R¹ group by the interconversion procedures described hereinabove. It may be necessary to protect the 3' and 4' hydroxyl groups when manipulating the 2'-axial hydroxyl group. Protection may conveniently be effected by the formation of an isopropylidene group using a procedure described in the first embodiment of process (A). Subsequent removal of this group to provide the diol function may be achieved by treatment with an acid such as an inorganic acid (e.g. hydrochloric acid).

When the carboxyl group in the compound of formula (IX) is protected with a diphenylmethyl group the protection reaction may conveniently be carried out by treating a solution of the compound of formula (IX) in an alcoholic solvent (e.g. methanol) and/or in a halogenated hydrocarbon (e.g. dichloromethane) with diphenyldiazomethane, conveniently added as a solution in a halogenated hydrocarbon solvent (e.g. dichloromethane).

Compounds of formula (II) wherein X and Y represent oxygen or sulphur may also be prepared by the decarbonylation of a corresponding protected derivative of a compound of formula (I) in which CR²R³ represents C=O and n is zero. The decarbonylation may conveniently be effected by addition of an alkoxy compound (e.g. CH₃ONa) in a suitable solvent such as an alcohol (e.g. methanol).

Compounds of formula (VII) may conveniently be prepared from sordaricin using conventional carboxyl group protecting means. Thus, for example, when R^{p} in a compound of formula (VII) represents trimethylsilylethyl this group may be introduced by treating sordaricin with O-[2-(trimethylsilyl)ethyl]-N,N'-diisopropylisourea, conveniently in a suitable solvent such as an ether (e.g. tetrahydrofuran) at an elevated temperature (e.g. under reflux). When R^{p} represents diphenylmethyl this group may be introduced using the methodology described above.

A compound of formula (VI) may conveniently be prepared from a corresponding compound of formula (III) by converting the appropriate hydroxyl group in (III) to an amino group by the following sequence of steps 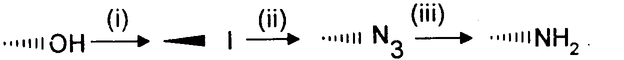
Step (i) may conveniently be carried out by the addition of iodine to a solution of a compound of formula (III) in a suitable solvent such as a hydrocarbon (e.g. toluene) in the presence of triphenylphosphine and imidazole, and then heating the mixture, e.g. under reflux.
Step (ii) may conveniently be carried out by adding an azide (e.g. lithium azide) and then heating the mixture, for example within the range of 40° to 80°C, in a suitable solvent such as an amide (e.g. dimethylformamide).
Step (iii) may conveniently be effected by treating the azide with hydrogen in the presence of a palladium catalyst (e.g. 10% palladium on charcoal) in a suitable solvent such as an ester (e.g. ethyl acetate) at about room temperature.

Compounds of formulae (II) to (VII) and (IX) are novel intermediates and form further individual aspects of the present invention. The compound of formula (IX), 4'-demethylsordarin, represents a particular aspect of the present invention.

Compounds of formula (VIII) are either known in the art or may be prepared by methods analogous to those used to prepare the known compounds of formula (VIII).

Base salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as an alcohol (e.g. methanol) or dioxane at temperatures of for example 0°C to 80°C and conveniently at about room temperature.

Pharmaceutically acceptable salts may also be prepared from other salts, including other pharmaceutically acceptable salts of the compounds of formula (I), using conventional methods.

The novel compound of formula (IX) may conveniently be prepared according to the fermentation process described hereinafter or by demethylating sordarin using a biotransformation procedure. It is to be understood that such processes for the preparation of the compound of formula (IX) represent further aspects of the present invention.

The fermentation process comprises cultivating a microorganism capable of producing the compound of formula (IX) and thereafter isolating the compound of formula (IX) from the culture.

Microorganisms capable of producing the compound of formula (IX) will conveniently be mutant strains of Sordaria araneosa which can readily be identified by screening survivors of mutagenesis by analysing a test sample obtained from fermentation of the microorganism using standard methodology. In particular, the microorganism to be conveniently used is a mutant strain of Sordaria araneosa deposited in the permanent culture collection of the CAB International Mycological Institute, Genetic Resource Reference Collection, Bakeham Lane, Egham, Surrey TW20 9TY, England. The strain was received by the Institute on 10 June, 1994 and was subsequently given the accession number IMI 362184 and dates of acceptance and confirmation of viability of 13 and 21 June, 1994 respectively. The Institute is an International Depository authority recognised under the Budapest Treaty. The characteristics thus far identified for IMI 362184 are given in Example 25.

The present invention provides in a further aspect the microorganism IMI 362184 per se and mutants thereof.

Mutants of the IMI 362184 may arise spontaneously or may be produced by a variety of methods including those outlined in Techniques for the Development of Microorganisms by H I Adler in "Radiation and Radioisotopes for Industrial Microorganisms", Proceedings of the Symposium, Vienna 1973, p241, International Atomic Energy authority. Such methods include ionising radiation, chemical methods, e.g. treatment with N-methyl-N'-nitro-N-nitrosoguanidine (NTG), heat, genetic techniques, such as recombination and transformation, and selective techniques for spontaneous mutants.

The preparation of the compound of formula (IX) by fermentation may be effected by conventional means i.e. by culturing a suitable organism in the presence of assimilable sources of carbon, nitrogen and mineral salts, and thereafter isolating the desired product.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium, potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20° to 40^{o}C, preferably from 20 to 35^{o}C, especially about 25°C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium. The fermentation will generally be carried out in the pH range 3.5 to 9.5, preferably 4.5 to 7.5. It may be necessary to add a base or an acid to the fermentation medium to keep the pH to within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for a period of 4-30 days, preferably about 5-15 days. An antifoam may be present to control excessive foaming and added at intervals as required. Carbon and/or nitrogen sources may also be fed into the fermentation medium as required.

The compound of formula (IX) is associated mainly with the cells and may be brought into solution either by addition of an acid and a water-miscible organic solvent, or more preferably by addition of a base (e.g. sodium hydroxide). Cells may be separated from these solutions either by centrifugation, conventional filtration or membrane filtration. The liquor may be optionally thereafter treated with an acid such as sulphuric acid until the pH is below 6 (e.g. about pH 4.5).

The compound of formula (IX) may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be particularly suitable for isolating and purifying the compound of formula (IX).

Suitable solid supports include silica; a non-functional macroreticular adsorption resin, for example cross-linked styrene divinyl benzene polymer resins such as CG161 and Amberlite XAD-2, XAD-4, XAD-16 or XAD-1180 resins (Rohm & Haas Limited) or Kastell S112 (Montedison); a substituted styrene-divinyl benzene polymer such as Diaion SP207 (Mitsubishi); an anion exchanger [e.g. IRA-958 or MacroPrep High Q (BioRad)], an organic solvent-compatible cross-linked dextran such as Sephadex LH20 (Pharmacia UK Limited), or on reverse phase supports such as hydrocarbon linked silica, e.g. C₁₈-linked silica.

The compound of formula (IX) may also be isolated and purified by the use of a liquid anion exchanger such as LA 2.

Suitable solvents for the elution of the compound of formula (IX) will, of course, depend on the nature of the adsorbent. When using a polymer resin such as XAD-16 water-miscible solvents such as methanol, acetone, isopropanol or acetonitrile in various proportions in water may be particularly suitable.

The presence of the compound of formula (IX) during the extraction/isolation procedures may be monitored by conventional techniques such as high performance liquid chromatography (HPLC) or UV spectroscopy or by utilising the optical rotation or other property of the compound.

Where the compound of formula (IX) is obtained in the form of a solution in an organic solvent, for example after purification by absorption/elution, the solvent may be removed by conventional procedures, e.g. by evaporation, to yield the required compound. If desired, the compound may be further purified by chromatographic techniques such as countercurrent chromatography using a coil extracter such as a multi-layer coil extracter or high performance liquid chromatography or supercritical fluid chromatography on adsorbents such as carbon, alumina, vanadium, polymeric resins or silica, with or without bonded phases. Suitable solvents/eluents for the chromatographic purification/separation of the compound of formula (IX) will of course depend on the nature of the adsorbent. When using a C8 bonded silica, mixtures of acetonitrile and water are particularly suitable. Alternatively, the compound may be further purified by solvent extraction, for example using an appropriate organic solvent such as a ketone (e.g. acetone or methyl ethyl ketone), a halogenated hydrocarbon, an alcohol (e.g. methanol), a diol (e.g. propane-1,2-diol or butane-1,3-diol) or an ester (e.g. methyl acetate or ethyl acetate) In a further alternative, solutions of compound (IX) may be further purified by treatment with adsorbents that selectively remove impurities when added at appropriate levels (e.g. DEAE-cellulose) or by crystallisation (e.g. from a mixture of acetonitrile and water) or using a combination of the above procedures.

The biotransformation of sordarin to 4'-demethylsordarin, the compound of formula (IX), may be effected by incubating sordarin in a culture comprising a suitable organism and sources of carbon and nitrogen, including those sources specified hereinabove, and thereafter isolating the compound of formula (IX) from the culture.

Microorganisms capable of demethylating sordarin at the 4'-position may readily be identified by using a small scale test and analysing a test sample obtained using standard methodology, for example, using HPLC. Examples of microorganisms which have been identified as sordarin demethylators include Streptomyces capreolus ATCC 31963, Streptomyces avermitilis ATCC 31272, Streptomyces armentosus NRRL 3176, Streptomyces antibioticus ATCC 31771, Streptomyces rimosus ATCC 23955, Streptomyces platensis ATCC 29778, Streptomyces mashuensis ATCC 23934, Streptomyces eurythermus ATCC 14975, Nocardia orientalis ATCC 43491 and Cunninghamella echinulata var elegans ATCC 36112.

Cultivation of the organism will generally be effected at a temperature of from 20° to 40°C, preferably from 20° to 35°C, especially about 28°C, and will desirably take place with aeration and agitation, e.g. by shaking or stirring. The medium may initially be inoculated with a small quantity of mycelium and/or spores. The vegetative inoculum obtained may be transferred to the fermentation medium or to one or more seed stages where further growth (e.g. over about 1-3 days) takes place before transfer to the principal fermentation medium. The principal fermentation medium will also comprise sordarin and the fermentation will generally be carried out in a pH range of 3.5 to 9.5, preferably 4.5 to 7.5. It may be necessary to add a base or an acid to the fermentation medium to keep the pH to within the desired range. Suitable bases which may be added include alkali metal hydroxides such as aqueous sodium hydroxide or potassium hydroxide. Suitable acids include mineral acids such as hydrochloric, sulphuric or phosphoric acid. Fermentation may be carried out over a period of 2 to 5 days, preferably about 3 days. An antifoam may be present to control excess foaming and added at intervals as required. Carbon and/or nitrogen sources may also be fed into the fermentation medium as required.

The separation and isolation of the compound of formula (IX) from the fermentation broth may be effected by the general procedures previously described. When it is desired to lower the pH of the liquor to below pH 6 (e.g. to about pH 2.5) this may conveniently be achieved by the addition of an acid such as orthophosphoric acid.

It will be appreciated that biotransformation may be effected according to a number of alternative methods. For example, cells may be grown and harvested prior to addition to a solution of sordarin in, for example, buffer, spent fermentation medium or water. It is also feasible that the appropriate enzymes could be isolated and used (with appropriate co-enzymes) or the enzymes cloned and over-expressed.

As stated hereinabove, sordarin and sordaricin are known compounds, which may be obtained using procedures described in the relevant art. Thus, for example, the preparation of sordarin by the cultivation of Sordaria araneosa NRRL 3196 (also deposited in the ATCC as ATCC 36386) is described in British Patent Specification No. 1,162,027. Specific examples of the preparation of sordarin using similar procedures are reported hereinafter.

Sordaricin may conveniently be prepared under fermentation conditions similar to those described for preparing sordarin using Sordaria araneosa NRRL 3196 or a suitable mutant thereof, with isolation of the desired compound using appropriate chromatographic means. One such mutant has been deposited in the permanent culture collection of the CAB International Mycological Institute, Genetic Resource Reference Collection, Bakeham Lane, Egham, Surrey TW20 9TY, England. The strain was received and accepted by the Institute on 11 August, 1994 and was subsequently given the accession number IMI 362947 and a date of confirmation of viability of 19 August, 1994. The Institute is an International Depository authority recognised under the Budapest Treaty. The characteristics thus far identified for IMI 362947 are given in Example 26.

The present invention provides in a further aspect the microorganism IMI 362947 per se and mutants therof.

Processes for obtaining mutants of IMI 362947 and its genetic material will be similar to those described hereinabove for the manipulation of IMI 362184.

Sordaricin may also be prepared from sordarin using a biotransformation procedure. The biotransformation may conveniently be effected by incubating sordarin in a culture comprising a suitable organism and sources of carbon and nitrogen, including those sources specified hereinabove, and thereafter isolating sordaricin from the culture.

Microorganisms capable of converting sordarin to sordaricin may readily be identified by using a small scale test and analysing a test sample obtained using standard methodology, for example, using HPLC. We have identified one such microorganism and deposited it with the National Collections of Industrial and Marine Bacteria Limited (NCIMB), 23 St. Machar Drive, Aberdeen AB2 1RY, Scotland. The strain was received by the NCIMB on 4 August, 1994 and was the same day accepted for deposit for patent purposes and the viability of the microorganism confirmed. The microorganism, which is a Coryneform species having the characteristics given in Example 27, has been assigned the accession number NCIMB 40675. The NCIMB is an International Depository authority recognised under the Budapest Treaty.

The invention thus provides in another aspect the microorganism NCIMB 40675 per se and mutants thereof.

According to another aspect of the present invention we provide the genetic material of NCIMB 40675 and mutants thereof that participates in the bioconversion of sordarin to sordaricin.

Processes for obtaining mutants of NCIMB 40675 and its genetic material will be similar to those described hereinabove for the manipulation of IMI 362184.

Cultivation of the NCIMB 40675 will generally be effected at a temperature of from 20° to 40°C, preferably from 20° to 35°C, especially about 28°C, and will desirably take place with aeration and agitation, e.g. by shaking or stirring. The medium may initially be inoculated with a small quantity of mycelium and/or spores. The vegetative inoculum obtained may be transferred to the fermentation medium or to one or more seed stages where further growth (e.g. over about 1-3 days) takes place before transfer to the principal fermentation medium. The principal fermentation medium will also comprise sordarin and the fermentation will generally be carried out in a pH range of 3.5 to 9.5, preferably about 7.5. It may be necessary to add a base or an acid to the fermentation medium to keep the pH to within the desired range. Suitable bases which may be added include alkali metal hydroxides such as aqueous sodium hydroxide or potassium hydroxide. Suitable acids include mineral acids such as hydrochloric, sulphuric or phosphoric acid. Fermentation may be carried out over a period of 4 to 8 days, preferably about 6 days. An antifoam may be present to control excess foaming and added at intervals as required. Carbon and/or nitrogen sources may also be fed into the fermentation medium as required.

It will be appreciated that biotransformation may be effected according to a number of alternative methods. For example cells may be grown and harvested prior to addition to a solution of sordarin in, for example, buffer, spent fermentation medium or water. It is also feasible that the appropriate enzymes could be isolated and used or the enzymes cloned and overexpressed.

The separation and isolation of sordaricin from the fermentation broth may be effected by the general procedures previously described. When it is desired to lower the pH of the liquor to about pH 6 this may conveniently be achieved by the addition of an acid such as orthophosphoric acid.

It is to be understood that the fermentation and bioconversion processes described hereinabove for preparing sordaricin represent further aspects of the present invention.

The examples hereinafter illustrate aspects of the present invention and are not intended to limit the invention in any way.

### PREPARATION 1

### Production of sordarin

A culture of *Sordaria araneosa* NRRL3196 (ATCC36386) was grown on an agar medium until mature growth occurred. 6mm diameter plugs of the agar containing the growth were transferred to sterile water or Brain Heart Infusion broth (Oxoid) + 10% glycerol and stored at ambient temperature or -140°C respectively. A suspension containing 2 of these agar plugs was used to inoculate a 250ml Erlenmyer flask containing 50ml of medium FS.

| Medium FS | g/L |
|---|---|
| Peptone(Oxoid L34) | 10 |
| Malt extract (Oxoid L39) | 21 |
| Glycerol (Glycerine CP) | 40 |
| Junlon 110 (Honeywell & Stein) | 1 |
| Distilled water | |

The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) which were incubated as described above. 80ml of the bulked shake flask developed inoculum was used to inoculate each of two 7L fermenters containing 5L of medium FS. The fermentations were controlled to a temperature of 25C. The culture was agitated at 400 rpm and aerated at 2 Lpm. After 3 days fermentation, 10L of culture was used to inoculate a 780L fermenter containing 500L of medium SM55VAR.

| SM55VAR | g/L |
|---|---|
| Glucidex 32D (Roquette Frere) | 74 |
| Peptone (Oxoid L37) | 10 |
| Proflo (Traders Protein) | 30 |
| Beet molasses | 15 |
| MgSO₄.7H₂O(BDH) | 5 |
| CaCO₃ (BDH) | 5 |
| FeSO₄.7H₂O (Sigma) | 2 |
| ZnSO₄.7H₂0 (BDH) | 0.04 |
| L-trytophan (Sigma) | 2 |
| PPG2000 (K & K Greef) | 0.5 |
| Silicone 1520 (Dow Corning) | 0.04 |
| Distilled water | |

The fermentation was controlled to a temperature of 25°C. The broth was agitated at 300-350 rpm and aerated at 200 Lpm. 70% (w/v) Meritose (Tunnel Refineries) solution was fed to the culture to maintain a positive residual glucose concentration. Distilled water was fed to maintain a culture volume of 500L. PPG2000 antifoam was added on demand to control foaming. Whole broth extracts (in aqueous acetonitrile + 1% trifluoroacetic acid) were assayed for presence of sordarin by reverse phase HPLC. The culture was harvested after 11 days when the extract of a broth sample indicated a sordarin titre of 0.6 g/L. Fermentation broth was made 0.1M with respect to sodium hydroxide and after storage at ambient temperature overnight was filtered through Dicalite on a rotary vacuum filter (1% Dicalite was added to the broth as filter aid). The filtrate was adjusted to pH 6-7 with concentrated sulphuric acid and the solution was applied to XAD16 resin (10 volumes of filtrate/volume resin). The adsorbent was washed with water and acetone:water (1:3) in both cases to give a clear effluent before sordarin was eluted with acetone:water (3:1 ; 2 column volumes collected). Flow rate throughout the process was between 1-2 column volumes/hr. The eluate was concentrated to a small volume (8.5L). The concentrate was adjusted to pH 3 with phosphoric acid and stood overnight at ambient temperature to allow precipitated sordarin to settle. Supernatant was decanted then centrifuged and the supernatant was discarded. Centrifuge pellets and precipitate were taken up in 75% aqueous acetonitrile to give 3.9L of a dark brown solution. To this was added 1.0L 0.2M NH₄H₂PO₄ with stirring, and the solution was adjusted to pH 4.0 with phosphoric acid, to give a final volume of 5.0L with approximate composition 60% acetonitrile - 0.1M NH₄H₂PO₄. The crude sordarin solution (5.0L) from above was subjected to preparative HPLC in 10 injections (450-550ml each) on a column (15cm x 25cm) of 7mm Kromasil C8 in mobile phase of 50% acetonitrile - 0.1M NH₄H₂PO₄, pH 4 (50L acetonitrile made up to 100L with water containing 575g NH₄H₂PO₄ and 40ml H₃PO₄), flow-rate 600ml/min, detection by UV absorbance (λ 210nm).
The fraction eluting between 15.4 and 19.2 min was collected. Pooled fractions from the 10 injections (23L) were diluted with water to 50L and this solution was pumped back through the Kromasil column at 28L/h. The column was washed with water (25L) then eluted with 90% acetonitrile (10L). The eluate was evaporated to a residue of 1300ml which was freeze-dried to yield the title compound as a buff powder (105.6g). MS and NMR analysis of the product showed equivalence with an authentic sample of sordarin.

### PREPARATION 2

### Production of sordarin potassium salt

*Sordaria araneosa* NRRL3196 (ATCC36386) was maintained in Brain Heart Infusion broth (Oxoid) + 10% glycerol at -140°C as described in Preparation 1. A suspension containing 2 agar plugs was used to inoculate a 250ml Erlenmyer flask containing 50ml of medium FS. The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) and incubated as described above. 80ml of the bulked shake flask developed inoculum was used to inoculate each of two 7L fermenters containing 5L of medium FS. The fermentations were controlled to a temperature of 25°C. The culture was agitated at 400 rpm and aerated at 2 Lpm. After 3 days fermentation, 10L of culture was used to inoculate a 780L fermenter containing 500L of medium SD1.

| SD1 | g/L |
|---|---|
| Meritose (Tunnel refineries) | 22 |
| Lactose | 20 |
| Glucidex 32D (Roquette Frere) | 30 |
| Arkasoy (The British Arkady Co.) | 20 |
| CSL | 20 |
| FeCl₃.6H₂O | 0.05 |
| NH₄H₂PO₄ | 5 |
| ZnSO₄.7H₂O (BDH) | 0.1 |
| PPG2000 | 0.5 |
| Distilled water | |

The fermentation was controlled to a temperature of 25°C. The broth was agitated at 350 rpm and aerated at 200 Lpm. Distilled water was fed to maintain a culture volume of 500L. Whole broth extracts were assayed for presence of sordarin by reverse phase HPLC. The culture was harvested after 6 days when the extract of a broth sample indicated a sordarin titre of 1.3 g/L. A 50L sample of harvest broth was made 0.1M with respect to sodium hydroxide and stored at 4°C overnight. Cells were removed by vacuum filtration through a bed of Dicalite adding an additional 2% Dicalite to the broth as filter aid. The filtrate was adjusted to approximately pH 6 with concentrated sulphuric acid. Broth extract was pumped through a bed of Amberchrom CG161 resin at 320ml/min (0.64 bed volumes per min). The effluent was monitored by HPLC. After 45 min (equivalent to a pumped volume of 14.4L) sordarin began to break through and pumping was halted. The adsorbent was washed with water (2L) then 25% v/v acetone in water (2L). Sordarin was eluted with 75% v/v acetone in water (1.5L). The 75% v/v acetone eluate was evaporated to about 200ml by rotary evaporation at 40°C. 200ml butan-1-ol was added and the evaporation continued until a viscous oil remained (containing some butan-1-ol). The oily residue was extracted with hot methanol (2 x 500ml). Extracts were combined, filtered (Whatman no 1 paper) then evaporated at 40 to 45°C to give a viscous oil. This was extracted with hot acetone (2 x 500ml). Acetone extracts were combined, filtered and evaporated to a viscous oil. Propan-2-ol (350ml) was added and the oil dissolved at 45°C to give a clear brown solution. A solution of potassium-2-ethylhexanoate (39% w/w solution in propan-2-ol, 54g) was weighed into a 500ml conical flask. The sordarin-containing solution in propan-2-ol was poured into the conical flask, the contents mixed, and left to stand for 4h at room temperature. The solution was seeded with sordarin potassium salt (about 5mg) and the stoppered flask was stored for 3 days at 4°C. The off-white solid which formed was filtered under vacuum (No 4 sinter funnel) and the filter cake was washed with propan-2-ol (about 20-30ml). The solid was transferred to a crystallising dish and dried *in vacuo* over P₂O₅ for 16h to give the title compound (10.5g).

### PREPARATION 3

### Production of sordarin potassium salt

500 litres of culture broth was prepared as in Preparation 1. The broth was made 0.1M with respect to sodium hydroxide and after 4 days at 0°C was filtered through a bed of Dicalite on a rotary vacuum filter. 1% Dicalite was added to the broth as filter aid. The pH of the filtrate was adjusted from pH 9.6 to pH 7.5 with concentrated sulphuric acid. Filtrate (10L) was adjusted to pH 6 with H₃PO₄ and applied to a bed (200ml) of XAD-16 resin packed in water at a flow-rate of 1-1.5 bed volumes/h. The resin bed was washed with water (1 bed volume) then with 25% aqueous isopropanol (2.5 bed volumes) before elution with neat isopropanol. After a fore-run of 50ml the isopropanol eluate was collected as 100ml fractions. Sordarin-rich fractions 2-6 inclusive were pooled and evaporated to half volume. Isopropanol was added to bring the volume back to 500ml, then the evaporation to half volume repeated to remove residual water by azeotropic distillation. After a third evaporation to half volume the residue was filtered and the filter was washed with isopropanol. To the combined filtrate and washings (400ml) was added a solution of potassium 2-ethylhexanoate (8g) in isopropanol (100ml). The mixture was seeded with sordarin potassium salt and stood for several days at 4°C whilst a slow crystallisation took place. Crystals were filtered on a No. 3 sinter, washed with a little ice-cold isopropanol and dried *in vacuo* to yield the title compound as a pale brown powder (4.85g).

### PREPARATION 4

### Production of 4'-demethylsordarin

(i) IMI 362184 was maintained in Brain Heart Infusion broth (Oxoid) + 10% glycerol at -140°C as described in Preparation 1. A suspension containing 2 agar plugs was used to inoculate a 250ml Erlenmyer flask containing 50ml of medium FS. The culture was incubated at 25°C for 5 days on a rotary shaker operated at 250 rpm with a 50mm diameter orbital motion. Aliquots (2ml) of the developed inoculum were used to inoculate further 250 ml Erlenmyer flasks containing medium FS (50ml) and incubated as described above. 80ml of the bulked shake flask developed inoculum was used to inoculate each of two 7L fermenters containing 5L of medium FS. The fermentations were controlled to a temperature of 25°C. The culture was agitated at 400 rpm and aerated at 2 Lpm. After 3 days fermentation, 10L of culture was used to inoculate a 780L fermenter containing 500L of medium SM55VAR (as described in Preparation 1). The fermentation was controlled to a temperature of 25°C. The broth was agitated at 350 rpm and aerated at 500 Lpm. 70% w/v Meritose (Tunnel Refineries) solution was fed to the culture to maintain a positive residual glucose concentration. Distilled water was fed to maintain a culture volume of 500L. Whole broth extracts were assayed for presence of 4'-demethylsordarin by reverse phase HPLC. The culture was harvested after 10 days when the extract of a broth sample indicated a 4'-demethylsordarin titre of 0.8 g/L. Fermentation broth was made 0.1M with respect to sodium hydroxide and after 1 hour at ambient temperature was filtered through Dicalite on a rotary vacuum filter (1% Dicalite was added to the broth as filter aid). The filtrate was adjusted to pH 4.5 with concentrated sulphuric acid and the solution was applied to XAD16 resin (20g product/L resin). The adsorbent was washed with 0.1% phosphoric acid (10 column volumes) and acetonitrile:water 1:4 (6 column volumes) before the product was eluted with acetonitrile:water (1:1 ; 2 column volumes). Flow rate throughout the process was between 1-2 column volumes/hr. The eluate was concentrated to dryness with the addition of butan-1-ol and the solid was extracted with methanol (12L) followed by acetone (10L) at 60°C. Insoluble material was removed at each stage by filtration through a no 3 glass sinter and the extracts concentrated to dryness as before. The solid was crystallised from acetonitrile:water (3:7) before being recrystallised from the same solvent and dried to constant weight over P₂O₅ to give the title compound (244.0g), which by proton NMR analysis showed equivalence with an authentic sample of 4'-demethylsordarin.
(ii) The fermentation procedure in (i) above was followed, and after the broth was made 0.1M with respect to sodium hydroxide this was ultrafiltered through ETNA 10A membrane (10kDalton cut-off). After diafiltration with water the bulked permeate was a clear solution. After adjustment to pH 5.2, using concentrated sulphuric acid, the permeate was loaded to a column of XAD16 resin at a rate of 2 column volumes per hour to give a final loading of 32g 4'-demethylsordarin per litre of resin. The column was washed at a rate of 2 column volumes per hour, first with 0.1% v/v phosphoric acid and then with 20% v/v acetonitrile/water (10 column volumes of each). The column was eluted with 65% acetonitrile/water at 2 column volumes per hour. A forerun of 0.75 column volumes was discarded. 85% of the loaded 4'-demethylsordarin was recovered in the next 1.6 column volumes of eluate. The rich eluate was treated by stirring for 5 minutes with 2% w/v of DE52 cellulose, which was removed by filtration. An aliquot of DE52 treated eluate was concentrated to 62% of the original volume (43% acetonitrile) using a rotary evaporator. The concentrated eluate was held at 4°C for 15 hours to crystallise and the solids formed collected by filtration through a glass sinter. The crystals were washed with four cake volumes of 30% v/v acetonitrile/water and dried at 30°C in vacuo. The title compound (1.94g) was recovered from the concentrated eluate as a pale grey solid.

### PREPARATION 5

### Screen for microorganisms capable of demethylating sordarin at the 4' position

Microorganisms capable of demethylating sordarin at the 4'-position could be identified by growing them at 28°C (bacteria) or 25°C (fungi) in 10ml volumes of SB1 (bacteria) or FB1 (fungi) in 50ml conical flasks shaken at 250rpm. After 2 days, sordarin was added to a final concentration of 0.5mg/ml (from a 200mg/ml stock solution in 80% ethanol) and the flasks were then incubated for a further 3 days. A 500µl sample of whole culture was mixed in an Eppendorf tube with 500µl of 80% acetonitrile/2% trifluoroacetic acid and left to extract at room temperature for 30 minutes. The extract supernatant, obtained by centrifuging samples in a microfuge was assayed by isocratic HPLC for the presence of 4'-demethylsordarin. 4'-demethyl sordarin eluted at 3.35mins with a mobile phase of 35% acetonitrile in water, flow rate 2ml/min, using a Spherisorb C₆ column (5µm, 15cm x 4.6mm). By this method, the following microorganisms were identified as sordarin demethylators:
- *Streptomyces capreolus*: ATCC 31963
- *Streptomyces avermitilis*: ATCC 31272
- *Streptomyces armentosus*: NRRL 3176
- *Streptomyces antibioticus*: ATCC 31771
- *Streptomyces rimosus*: ATCC 23955
- *Streptomcyes platensis*: ATCC 29778
- *Streptomyces mashuensis*: ATCC 23934
- *Streptomyces eurythermus*: ATCC 14975
- *Nocardia orientalis*: ATCC 43491
- *Cunninghamella echinulata var elegans*: ATCC 36112

| SB1 Medium | g/L |
|---|---|
| Arkasoy | 25 |
| Yeast extract | 5 |
| KH₂PO₄ | 5 |
| Glucose | 20 |
| Distilled Water | |
| pH 7 | |

| FB1 Medium | |
|---|---|
| Soya oil | 30 |
| Arkasoy | 10 |
| Yeast extract | 5 |
| K₂HPO₄ | 5 |
| Glucose | 20 |
| Distilled Water | |
| pH 5.5 | |

### PREPARATION 6

### Production of 4'-demethylsordarin by biotransformation of sordarin

0.3ml of a spore suspension of *Streptomyces capreolus* ATCC 31963 (in 15% v/v glycerol stored at -20°C) was inoculated into 30ml SB1 medium in a 250ml Erlenmeyer flask to give a seed culture which was incubated at 28°C and 250rpm on a rotary shaker. After 4 days, 0.5ml of this was used to inoculate 35ml SB1 in a 250ml flask which was grown for 48 hours at 28°C, 250rpm. At this stage, the culture was aliquoted as 10ml amounts into 50ml Erlenmeyer flasks which were fed with 5mg sordarin (from a 200mg/ml stock solution in ethanol). Incubation was continued for a further 3 days. 80% v/v acetonitrile in water (14ml) was added to whole broth (14ml) and the mixture was kept at room temperature and occasionally agitated. After 30 minutes, the cells were removed by centrifugation. Acetonitrile was removed by evaporation and the pH of the aqueous solution was adjusted to 2.5 with orthophosphoric acid. The solution was passed through a column containing Amberlite XAD-16 resin (bed volume 5ml). The adsorbent was washed sequentially with water (10ml), 10% v/v acetonitrile in water (20ml), 30% v/v acetonitrile in water (10ml), 50% v/v acetonitrile in water (20ml) and 90% v/v acetonitrile in water (10ml). Fractions were monitored by HPLC; 4'-demethylsordarin was located in the 50% v/v acetonitrile in water eluate. The fraction containing 4'-demethylsordarin was evaporated to dryness *in vacuo* at the room temperature and the residue was re-dissolved in 35% v/v acetonitrile in water (15ml). 4'-demethylsordarin was purified by preparative HPLC:

| | |
|---|---|
| Column | Spherisorb 5 micron C₆ 25cm x 2.5cm |
| Flow rate | 25ml/min |
| Detection | UV at 210nm |
| Mobile Phase | 350ml acetonitrile made up to 1000ml with 0.05M ammonium dihydrogen phosphate in water. pH adjusted to 2.5 with orthophosphoric acid |
| Injection volume | 4.5ml |

4'-Demethylsordarin was eluted after 10.0 minutes under these conditions. The pooled fractions from four HPLC runs were diluted 1:1 with water then pumped back onto the silica (after washing this with acetonitrile and re-equilibrating with water). The adsorbent was washed with water (200ml) and adsorbed product was eluted with 95% v/v acetonitrile in water (200ml). The acetonitrile/water eluate was evaporated to remove organic solvent, and the aqueous solution freeze dried to yield 4'-demethylsordarin (1.5mg) as a white powder.
δ (¹H,CDCl₃); 9.74(s,1H); 6.08(brd,3,1H); 4.70(d,1.5,1H); 4.16(d,9.5,1H); 4.08(dd,4.5,3.5,1H); 3.88(dd,4.5,1.5,1H); 3.75(dq,8.5,6,1H); 3.62(d,9.5,1H); 3.68(dd,8.5,3.5,1H); 2.65(m,1H); 2.34(m,1H); 1.32(d,6,3H); 1.30(d,12.5,1H); 1.23(m,1H); 1.04(d,7,3H); 0.99(d,7,3H); 0.81(d,7,3H)

### PREPARATION 7

### Production of sordaricin

The procedure according to Preparation 1 was followed up to and including the preparative HPLC stage. The fraction eluting between 21.4 and 25.0 min was collected. Pooled fractions from the 10 injections (22L) were diluted with an equal volume of water and this solution was pumped back through the Kromasil column at 28L/h. The column was washed with water (20L) then eluted with 90% acetonitrile (4.5L). This eluate was combined with the corresponding eluate fraction (4.5L) from a similar fermentation which had been processed by the same procedure. Combined 90% acetonitrile eluates were concentrated by rotary evaporation until product began to crystallise (volume about 1.6L). The residue was heated on a 60°C water bath with addition of the minimum volume of acetonitrile to give a clear solution. The solution was cooled and stored at 4°C. Crystals were filtered on a No.3 sinter and dried *in vacuo* to yield a brown solid. This was recrystallised from acetonitrile:water (40:60) and the product was filtered, washed with 25% acetonitrile and dried *in vacuo* to give the title compound (10.7g). MS and NMR analysis of the product showed equivalence with an authentic sample of sordaricin.

### PREPARATION 8

### Biotransformation of sordarin to sordaricin

A 0.2ml suspension of NCIMB 40675 was used to inoculate a 250ml Erlenmyer flask containing 50ml of nutrient broth (Oxoid) supplemented with 0.2% yeast extract. The culture was incubated at 28°C for 29 hours on a rotary shaker operated at 250rpm with a 50mm orbital motion. Aliquots (1ml) of the developed inoculum were used to inoculate further 250ml Erlenmyer flasks containing 50ml of double strength nutrient broth (Oxoid) supplemented with 0.1% yeast extract. These were incubated as above for 24 hours. 62 of these flasks were pooled to provide 2.65 litres of developed culture which was added to 30 litres sordarin fermentation filtrate, pH adjusted to 7.5, prepared as in Preparation 1. The reaction was carried out in a 70 litre fermenter at 30°C, agitated at 200rpm, aeration at 0.5VVM. The pH was controlled to 7.5 by addition of 1N hydrochloric acid. After 6 days, approximately 16g of sordarin was converted to sordaricin. Biotransformation broth was filtered through Dicalite on a vacuum filter and the bed was washed with water to give 31.5L filtrate, pH8.2. This was adjusted to pH6.0 with H₃PO₄ and pumped through a bed (25cmx5cm) of Amberchrom CG161 resin at 290ml/min. The Amberchrom bed was washed with 0.1% H₃PO₄ (2L) and 25% acetonitrile (4L) then eluted with 60% CH₃CN (2L). Eluate was concentrated by rotary evaporation until the onset of crystallisation, then the residue was heated on a 60°C water bath with additions of the minimum acetonitrile to give a clear solution. This was cooled and chilled at 4°C overnight. Crystals were filtered, washed with 25% acetonitrile and dried *in vacuo* to give a brown powder. Crude product was recrystallised from 1L of acetonitrile:water (40:60), filtered, washed and dried as above to yield sordaricin as pale brown needles (6.15g).

### PREPARATION 9

### Biotransformation of sordarin to sordaricin

A loopful of surface growth from an agar culture of NCIMB 40675 was used to inoculate a 250ml Erlenmeyer flask containing 50ml of nutrient broth (Oxoid) supplemented with 0.2% yeast extract. The culture was incubated for 26 hours at 25°C on a rotary shaker operating at 250rpm with a 50mm diameter throw. 1ml aliquots of the developed culture were used to inoculate 250ml Erlenmyer flasks as described above. The flasks were supplemented with pure sordarin in 80% ethanol to give a final concentration of 1.25mg/ml. The flasks were incubated as above for 8 days, then bulked to give 365mls of culture broth containing sordaricin. Bioconversion mixture was centrifuged to remove NCIMB 40675 cells, and supernatant (350ml) decanted. To supernatant was added Whatman Partisil P40 ODS-3 (5ml, pre-wetted with acetonitrile) and the pH of this mixture was adjusted to 4.0 with H₃PO₄. Partisil was filtered off on a Buchner funnel and eluted with acetonitrile (100ml) followed by 75% acetonitrile (100ml). Combined eluates were evaporated to an aqueous residue of 10-15ml. This was heated on a 60°C water bath and acetonitrile added until a clear solution was obtained, then heating maintained and water added until the solution became cloudy. The mixture was cooled and stored at 4°C for several days. Crystals were filtered on a No.3 sinter, washed with water and dried *in vacuo* over P₂O₅ to give sordaricin as white needles (92mg).

### PREPARATION 10

### Production of sordaricin

A frozen ampoule of IMI 362947 was used to inoculate 50ml of seed medium FS in a 250ml Erlenmeyer flask. This flask was incubated at 25°C for 6 days on a rotary shaker operated at 250rpm with a 50mm diameter orbital action. Aliquots (1ml) of the developed inoculum were used to inoculate four 250ml Erlenmeyer flasks containing 50ml shake-flask production medium SM55/A.

| SM55/A | g/l |
|---|---|
| Maltodextrin MD30E (Roquette Frere) | 120 |
| Beet molasses | 15 |
| Peptone (Oxoid L37) | 10 |
| Proflo (Traders Protein) | 30 |
| L-Tryptophan (Sigma) | 2 |
| ZnSO₄.7H₂O (BDH) | 0.04 |
| FeSO₄.7H₂O (Sigma) | 2 |
| CaCO₃(BDH) | 5 |
| MgSO₄.7H₂O(BDH) | 5 |
| Made up in distilled water and autoclaved at 121°C for 120 minutes. | |

These cultures were incubated as described above for 7 days. The flask contents were pooled and a 40ml portion was treated with 1N sodium hydroxide (4ml) with occasional agitation at room temperature for 45 minutes. The cells were then removed by centrifugation at 3000rpm for 20 minutes, the supernatant was taken and the pH was adjusted to 3 with 1N hydrochloric acid. The solution was passed through a Bond Elut column (1g size; C18) and the adsorbent was washed with water (20ml) then eluted with 90% v/v acetonitrile in water (15ml). The eluate was evaporated to dryness at room temperature and the residue was taken up in 50% v/v acetonitrile in water (9ml). Sordaricin was purified by preparative HPLC:

| | |
|---|---|
| Column | Spherisorb 5 micron ODS-2 (25cm x 2.1cm) |
| Flow rate | 25ml/min |
| Mobile phase | 550ml acetonitrile made up 1000ml with water, with 1ml trifluoracetic acid added per litre of mobile phase |
| Detection | 210nm |
| Injection volume | 4.5ml |

Sordaricin eluted after 6.0 minutes under these conditions. Fractions containing sordaricin were pooled, acetonitrile was removed by rotary evaporation at room temperature, and the aqueous solution was freeze dried to yield the title compound (8.9mg) as a white powder.

### INTERMEDIATE 1

### [1R-(1α, 3aβ,4β,4aβ,7β,7aα,8aβ)] 8a-[(6-Deoxy-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of 4'-demethylsordarin (10g) in methanol (200ml) was added dropwise at room temperature a 0.35M solution of diphenyldiazomethane (90ml) in methylene chloride, and the mixture was stirred for 6 hours. The solvent was evaporated to dryness and the residue chromatographed in a silica gel flash column with hexane:ethyl acetate (3:1) as eluent to give the title compound (12.6g) as a pale yellow foam.
δ (¹H, CDCl₃): 9.73 (s, 1H, CHO), 6.98 (s, 1H, CHPh₂), 6.05 (dd, 1H, H-2, J=1.5 and 3.6 Hz), 4.65 (d, 1H, H-1', J=1.5 Hz), 4.09, 3.76 (2d, 2H, 8a-CH₂, J=9Hz), 4.01 (m, 1H, H-2'), 3.84 (m, 1H, H-3'), 3.75 (m, 1H, H-5'), 3.69 (m, 1H, H-4'), 2.73 (t, 1H, H-1, J=4.2 Hz).

### INTERMEDIATE 2

### [1R-(1α, 3aβ, 4β, 4aβ, 7β, 7aα, 8aβ)] 8a-[(6-Deoxy-3,4-O-isopropylidene-β-D-altropyranosyloxy)methyl]-4-formyl-4,4a,5,6,7,7a,8,8a-octahydro-7-methyl-3-(1-methylethyl)-(1H)-1,4-methano-s-indacene-3a(1H)-carboxylic acid, diphenylmethyl ester

To a solution of Intermediate 1 (650mg) in 15ml of dimethoxypropane:acetone (1:2) was added *p*-toluensulphonic acid (10mg). The solution was stirred at room temperature for 1.5 hours, then potassium carbonate (1.0g) was added, the stirring continued for 30 minutes and the solvent evaporated to dryness. The crude mixture was partitioned between ethyl acetate (50ml) and water (25ml), the aqueous phase was extracted with ethyl acetate (2x50ml), the organic phase was washed with brine, dried over magnesium sulphate and evaporated to dryness. The residue was flash chromatographed on silica gel eluting with ethyl acetate:hexane (1:3) to give the title compound (600mg) as a white foam.
δ (¹H, CDCl₃): 9.73 (s, 1H, CHO), 7.45-7.24 (m, 10H, 2Ph), 6.98 (s, 1H, CHPh₂), 6.06 (dd, 1H, H-2, J=1.5 and 3.3 Hz), 4.57 (d, 1H, H-1', J=3.0 Hz), 4.30 (dd, 1H, H-3', J=3.6 and 5.7 Hz), 4.07 (d, 1H, 8aCH₂, J=9.0 Hz), 3.95-3.93 (m, 1H, H-2'), 3.85 (dd, 1H, H-4', J=5.7 and 9.3 Hz), 3.75 (d, 1H, 8aCH₂, J=9.0 Hz), 3.44 (dq, 1H, H-5', J_{d}=9.3 Hz, J_{q}=6.3 Hz), 2.73 (t, 1H, H-1, J=3.6 Hz).

### INTERMEDIATE 3

### [1R-(1α, 3aβ, 4β, 4aβ, 7β, 7aα, 8aβ)] 8a-[(6-Deoxy-3,4-O-(2-pentylidene)-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a suspension of Intermediate 1 (500mg) and *p*-toluensulphonic acid (5mg) in dichloromethane (5ml) was added 2,2-dimethoxypentane (2ml). The mixture was stirred at room temperature for 2 hours. Potassium carbonate (150mg) was added and the stirring continued for 30 minutes. The solvent was evaporated to dryness and the residue flash chromatographed on silica gel eluting with ethyl acetate:hexane (15:85) and (30:70) to give the title compound (474mg).
δ (¹H, CDCl₃): 9.73 (s, 1H, CHO), 7.45-7.24 (m, 10H, 2Ph), 6.98 (s, 1H, CHPh₂), 6.06 (dd, 1H, H-2, J=1.5 and 3.3 Hz), 4.57 (d, 1H, H-1', J=2.1 Hz), 4.30 (dd, 1H, H-3', J=3.9 and 6.0 Hz), 4.04 ( d, 1H, 8aCH₂, J=9.0 Hz), 3.94-3.91 (m, 1H, H-2'), 3.86 (dd, 1H, H-4', J=6.3 and 9.6 Hz), 3.73 (d, 1H, 8aCH₂, J=9.0 Hz), 3.45 (dq, 1H, H-5, J_{d}= 9.0 Hz, J_{q}=6.0 Hz), 2.72 (t, 1H, H-1, J=3.6 Hz).

### INTERMEDIATE 4

### [1R-(1α, 3aβ, 4β, 4aβ, 7β, 7aα, 8aβ)] 8a-[(6-Deoxy-3,4-O-(4-methoxy-2-butylidene)-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a suspension of Intermediate 1 (350mg) and *p*-toluensulphonic acid (5mg) in dichloromethane (4ml) was added 2,2,4-trimethoxybutane (1ml). The mixture was stirred at room temperature for 1 hour. Potassium carbonate (150mg) was added and the stirring continued for 30 minutes. The solvent was evaporated to dryness and the residue flash chromatographed on silica gel eluting with ethyl acetate:hexane (3:7) and (4:6) to obtain the title compound (290mg) in a 3:1 epimer ratio.
δ (¹H, CDCl₃) signals of the major component: 9.73 (s, 1H, CHO), 7.45-7.20 (m, 10H, 2Ph), 6.98 (s, 1H, CHPh₂), 6.06 (dd, 1H, H-2, J=1.5 and 3.6 Hz), 4.57 (d, 1H, H-1', J=2.1 Hz), 4.31 (dd, 1H, H-3', J=3.9 and 6.0 Hz), 4.05 (d, 1H, 8aCH₂, J=9.0 Hz), 3.95-3.90 (m, 1H, H-2'), 3.88 (dd, 1H, H-4', J=6.0 and 9.3 Hz), 3.73 (d, 1H, 8aCH₂, J=9.0 Hz), 3.51 (t, 2H, CH₂O, J=7.2 Hz), 3.50-3.45 (m, 1H, H-5'), 3.33 (s, 3H, CH₃O), 2.72 (t, 1H, H-1, J=3.6 Hz).

### INTERMEDIATE 5

### [1R-(1α, 3aβ, 4β, 4aβ, 7β, 7aα, 8aβ)] 8a-[(6-Deoxy-3,4-O-cyclopentylidene-β-D-altropyranosyloxy)]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a suspension of Intermediate 1 (160mg) in dichloromethane:1,1-dimethoxycyclopentane (3:1, 4ml) was added *p*-toluensulphonic acid (5mg) and the mixture was stirred at room temperature for 2 hours. Potassium carbonate (1g) was added and the stirring continued for 30 minutes. The solvent was evaporated to dryness and the residue was flash chromatographed on silica gel eluting with ethyl acetate:hexane (1:4) to obtain the title compound (142mg).
δ (¹H, CDCl₃): 9.73 (s, 1H, CHO), 7.45-7.25 (m, 10H, 2Ph), 6.98 (s, 1H, CHPh₂), 6.06 (dd, 1H, H-2, J=1.5 and 3.6 Hz), 4.55 (d, 1H, H-1', J=1.8 Hz), 4.17 (dd, 1H, H-3', J=3.0 and 5.1 Hz), 4.08 (d, 1H, 8aCH₂, J=9.0 Hz), 3.95 (dt, 1H, H-2', J_{d}=1.8 Hz, Jₜ=3.0 Hz), 3.81 (dd, 1H, H-4', J=5.1 and 9.3 Hz), 3.76 ( d, 1H, 8aCH₂, J=9.0 Hz), 3.42 (dq, 1H, H-5', J_{d}=9.3 Hz, J_{q}=6.3 Hz), 2.73 (t, 1H, H-1, J=3.9 Hz).

### INTERMEDIATE 6

### [1R-(1α, 3aβ, 4β, 4aβ, 7β, 7aα, 8aβ)] 8a-[(6-Deoxy-3,4-O-isopropylidene-2-O-(methylthio)thiocarbonyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

Intermediate 2 (100mg) and imidazole (1mg) were dissolved in dry tetrahydrofuran (4ml) under nitrogen atmosphere. Sodium hydride (5mg) was added and the suspension was stirred at room temperature for 0.5 hours. Carbon disulfide (27µl) was added, the stirring continued for 20 minutes and methyl iodide (18µl) was added. After 2 hours the reaction was stopped by addition of 1N ammonium chloride (20ml). The mixture was extracted with ethyl acetate (3x25ml), the organic phase was washed with brine, dried over magnesium sulphate and the solvent evaporated to dryness. The residue was purified in a flash chromatography column on silica gel eluting with ethyl acetate:hexane (1:9) to give the title compound (110mg).
δ (¹H, CDCl₃): 9.71 (s, 1H, CHO), 7.44-7.25 (m, 10H, 2Ph), 6.96 (s, 1H, CHPh₂), 6.01 (dd, 1H, H-2, J=1.2 and 3.6 Hz), 6.90 (dd, 1H, H-2', J=2.4 and 5.4 Hz), 4.85 (d, 1H, H-1', J=2.4 Hz), 4.53 (dd, 1H, H-3', J=5.4 and 6.3 Hz), 4.00 (dd, 1H, H-4', J=6.3 and 8.7 Hz), 3.93 (d, 1H, 8aCH₂, J=9.3 Hz), 3.65 (dq, 1H, H-5', J_{d}=8.7, J_{q}= 6.3 Hz), 2.68 (t, 1H, H-1, J=3.9 Hz), 2.59 (s, 3H, CH₃S).

### INTERMEDIATE 7

### [1R-(1α, 3aβ, 4β, 4aβ, 7β, 7aα, 8aβ)] 8a-[(2,6-Dideoxy-3,4-O-isopropylidene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

(i) Intermediate 6 (95mg) was dissolved in dry toluene (5ml) under nitrogen atmosphere and heated at 110^{o}C. A solution of tributyltin hydride (64µl) in dry toluene (5ml) was added dropwise over 1.5 hours with stirring. The heating was continued for another 1.5 hours, methanol (2ml) was added and the solvent evaporated to dryness. Flash chromatography of the residue on silica gel eluting with ethyl acetate:hexane (1:9) gave the title compound (42mg).
   δ (¹H, CDCl₃): 9.73 (s, 1H, CHO), 7.44-7.25 (m, 10H, 2Ph), 6.98 (1H, s, CHPh₂), 6.05 (dd, 1H, H-2, J=1.2 and 3.3 Hz), 4.54 ( dd, 1H, H-1', J=2.7 and 9.3 Hz), 4.39 (dt, 1H, H-3', J_{d}=2.7 Hz, Jₜ=3.6 Hz), 4.04 (d, 1H, 8aCH₂, J=9.0 Hz),3.67 (d, 1H, 8aCH₂, J=9.0 Hz), 3.65 (dd, 1H, H-4', J=3.6 and 8.7 Hz), 3.44 (dq, 1H, H-5', J_{d}=6.3 Hz, J_{q}=8.7 Hz), 2.75 (t, 1H, J=3.9 Hz).
(ii) A solution of tributyltin hydride (5.5ml) in dry toluene (150ml) was degassed with an argon stream for 1 hour and heated to reflux. A solution of Intermediate 6 (6g) in dry toluene (50ml) was then added over a period of 2 hours. Heating was continued until the reaction was complete (1.5 hours). Elimination of the solvent under reduced pressure gave a crude which was flash chromatographed over silica gel eluting with hexane and hexane:ethyl acetate (20:1 to 15:1) to afford the title compound (4g).

### INTERMEDIATE 8

### [1R-(1α, 3aβ, 4β, 4aβ, 7β, 7aα, 8aβ)] 8a-[(2,6-Dideoxy-3,4-O-(4-methoxy-2-butylidene)-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 4 (600mg) in dry tetrahydrofuran (10ml) at 0^{o}C and under nitrogen atmosphere were added sodium hydride (30mg) and imidazole (5mg). The solution was stirred for 10 minutes and carbon disulfide (147µl) was added. After 20 minutes methyl iodide (127µl) was added and the stirring continued for 30 minutes. 1N Ammonium chloride (20ml) was added and the mixture extracted with ethyl acetate (3x25ml), the organic phase washed with brine, dried over magnesium sulphate and the solvent evaporated to dryness. The residue was flash chromatographed on silica gel eluting with ethyl acetate:hexane (15:85) to give a white foam. This foam was dissolved in dry toluene (10ml) under nitrogen atmosphere and a solution of tributyltin hydride (0.46ml) in toluene (10ml) was added dropwise over 2 hours at 115°C. The heating was continued for 2 hours. The solvent was evaporated to dryness and the residue purified by flash chromatography on silica gel eluting with ethyl acetate:hexane (15:85) to give the title compound (260mg) in a epimer ratio of 4:1.
δ (¹H, CDCl₃): 9.63 (s, 1H, CHO), 7.44-7.20 ( m, 10H, 2Ph), 6.99 (s, 1H, CHPh₂), 6.06 (dd, 1H, H-2, J=1.2 and 3.3 Hz), 4.53 (dd, 1H, H-1', J=2.7 and 8.7 Hz), 4.42 (dt, 1H, H-3', J_{d}=3.0 Hz, J_{f}=4.2 Hz), 4.03 (d, 1H, 8aCH₂, J=9.0 Hz), 3.68 (dd, 1H, H-4', J=5.1 and 9.3 Hz), 3.66 (d, 1H, 8aCH₂, J=9.0 Hz), 3.52 (t, 2H, CH₂O, J=7.2 Hz), 3.40 (dq, 1H, H-5', J_{d}=9.3 Hz, J_{q}=6.3 Hz), 3.34 (s, 3H, CH₃O), 2.74 (t, 1H, H-1, J=4.2 Hz).

### INTERMEDIATE 9

### [1R-(1α, 3aβ, 4β, 4aβ, 7β, 7aα, 8aβ)] 8a-[(6-Deoxy-3,4-O-thiocarbonyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A mixture of Intermediate 1 (0.500g) and thiocarbonyldiimidazole (0.270g) in tetrahydrofuran (10ml) was refluxed for 6 hours. The solvent was evaporated in vacuo to give a yellow residue which was chromatographed on silica gel flash column eluting with hexane:ethyl acetate (3:1) to give the title compound (0.263g).
δ (¹H, CDCl₃): 9.71 (s, 1H, CHO), 7.44-7.26 (m, 10H, 2xPh), 6.97 (s, 1H, CO₂CHPh₂), 6.06 (dd, 1H, H-2, J=1.5 and 3.3 Hz), 4.93 (dd, 1H, H-3', J=3.9 and 7.2 Hz), 4.63 (d, 1H, H-1', J=2.1 Hz), 4.61 (dd, 1H, H-4', J=7.2 and 9.3 Hz), 4.12 (m, 1H, H-2'), 4.08 (d, 1H, 8aCH₂, J_{AB}=9.3 Hz), 3.74 (d, 1H, 8aCH₂, J_{AB}=9.3 Hz), 3.64 (m, 1H, H-5'), 2.69 (m, 1H, H-1).

### INTERMEDIATE 10

### [1R-(1α, 3aβ, 4β, 4aβ, 7β, 7aα, 8aβ)] 8a-[(2,6-Dideoxy-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 7 (1.5g) in a mixture of tetrahydrofuran (30ml) and methanol (15ml) was added dropwise at room temperature a 1N solution of hydrochloric acid (15ml) with vigorous stirring. Once the reaction was concluded (TLC control), saturated sodium bicarbonate (50ml) and ethyl acetate (200ml) were added and the mixture partitioned. The organic layer was washed with water (2x100ml) and dried over magnesium sulfate. Elimination of the solvent gave a residue which was flash chromatographed over silica gel eluting with hexane:ethyl acetate (5:1) and (2:1) to give the title compound (1.1g) as a white foam.
δ (¹H, CDCl₃): 9.73 (s, 1H, CHO), 7.5-7.2 (m, 10H, 2Ph), 6.99 (s, 1H, CHPh₂), 6.05 (dd, 1H, H-2, J=1.2 and 3.3 Hz), 4.64 (dd, 1H, H-1', J=2.1 and 9.6 Hz), 4.11 (m, 1H, H-3'), 4.06 (d, 1H, 8aCH₂, J_{AB}=9.3 Hz), 3.70 (m, 2H, H-5' and 8aCH₂), 3.34 (m, 1H, H-4'), 2.75 (t, 1H, H-1, J=3.6 Hz).

### INTERMEDIATE 11

### [1R-(1α, 3aβ, 4β, 4aβ, 7β, 7aα, 8aβ)] 8a-[(2,6-Dideoxy-3,4-O-thiocarbonyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid diphenylmethyl ester

A suspension of Intermediate 10 (160mg) and dibutyltin oxide (124.5mg) in dry toluene (15ml) was heated under reflux for 2 hours in a flask fitted with a Dean-Stark condenser and then allowed to stand at room temperature under nitrogen atmosphere. To the resulting solution was added phenyl-chlorothionoformate (69µl) in dry toluene (5ml). TLC control showed that the reaction was complete after 6 hours. The solvent was removed under reduced pressure and the crude mixture was flash chromatographed over silica gel eluting with hexane:ethyl acetate (20:1) and (15:1) to give the title compound (150mg) as a colourless oil.
δ (¹H, CDCl₃): 9.72 (s, 1H, CHO), 7.5-7.2 (m, 10H, 2Ph), 6.97 (s, 1H, CHPh₂), 6.05 (dd, 1H, H-2, J=1.5 and 3.6 Hz), 5.10 (m, 1H, H-3'), 4.61 (dd, 1H, H-1', J=2.4 and 8.4 Hz), 4.44 (dd, 1H, H-4', J=6.6 and 9.0 Hz), 4.04 and 3.67 (2d, 2H, 8aCH₂, J_{AB}=9 Hz), 3.62 (m, 1H, H-5'), 2.70 (t, 1H, H-1, J=2.0 Hz).

### INTERMEDIATE 12

### [1R-(1α,3aβ,4β,4aβ,7β,7aα,8aβ)] 8a-[(4-O-Allyl-2,6-dideoxy-β-D-allopyranosyloxy)methyl]-4-formyl-4-4a,5,6,7,7a,8,8a-octahydro-7-methyl-3-(1-methylethyl)-1,4-methano-s-indacene-3a(1H)-carboxylic acid, diphenylmethyl ester

A suspension of Intermediate 10 (400mg) and tributyltin oxide (240mg) in dry toluene (50ml) was heated to reflux in a flask fitted with a Dean-Stark condenser and then allowed to stand at room temperature under nitrogen atmosphere (approximately 10ml of azeotropic mixture was removed). Allyl bromide (71µl) and tetrabutylammonium fluoride (1 M solution in tetrahydrofuran, 0.95ml) were added consecutively and the mixture heated at 50^{o}C for 24 hours. Elimination of solvent gave a residue which was flash chromatographed over silica gel eluting with acetone:hexane (1:20) and (1:15) to give the title compound (300mg) as a colourless oil.
δ (¹H, CDCl₃): 9.72 (s, 1H, CHO), 7.52-7.2 (m, 10H, 2Ph), 6.98 (s, H, CHPh₂), 6.05 (dd, 1H, H-2, J=1.5 and 3.6 Hz), 5.89 (m, 1H, CH=CH₂), 5.30-5.20 (m, 2H, CH=CH₂), 4.64 (dd, 1H, H-1', J=2.1 and 9.6 Hz), 4.19 (m, 1H, H-3'), 4.15-3.95 (m, 3H, CH₂CH=CH₂+8aCH₂), 3.80-3.60 (m, 2H, H-5'+8aCH₂), 3.06 (dd, 1H, H-4', J=3.3 and 9.3 Hz), 2.75 (t, 1H, H-1, J=3.9 Hz), 2.38 (bd, 1H, OH).

### INTERMEDIATE 13

### [1R-(1α, 3aβ, 4β, 4aβ, 7β, 7aα, 8aβ)] 8a-[(2,6-Dideoxy-4-O-(2-methyl-2-propenyl)-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A suspension of Intermediate 10 (500mg) and tributyltin oxide (300mg) in dry toluene (50ml) was heated to reflux in a flask fitted with a Dean-Stark condenser and then allowed to stand at room temperature under nitrogen atmosphere (approximately 10ml of azeotropic mixture was removed). 3-Bromo-2-methylpropene (202µl) and tetrabutylammonium fluoride (1M solution in tetrahydrofuran, 0.90ml) were added consecutively and the mixture heated at 50^{o}C for 24 hours. Elimination of the solvent gave a residue which was flash chromatographed over silica gel eluting with acetone:hexane (1:20) and (1:15) to give the title compound (340mg) as a colourless oil.
δ (¹H, CDCl₃): 9.73 (s, 1H, CHO), 7.50-7.20 (m, 10H, 2Ph), 6.98 (s,1H, CHPh₂), 6.05 (dd, 1H, H-2, J=1.5 and 3.6 Hz), 4.96 and 4.91 (2m, 2H, =CH₂), 4.65 (dd, 1H, H-1', J=1.8 and 9.6 Hz), 4.20 (m, 1H, H-3'), 4.05 and 3.68 (2d, 2H, 8aCH₂, J_{AB}=9.3 Hz), 4.00 and 3.87 (2d, 2H, OCH₂, J_{AB}=12 Hz), 3.75 (m, 1H, H-5'), 3.06 (dd, 1H, H-4', J=3 and 9.6 Hz), 2.75 (t, 1H, H-1, J=3.6 Hz), 2.37 (bd, 1H, OH), 1.76 (bs,3H, CH₃C=CH₂).

### INTERMEDIATE 14

### [1R-(1α, 3aβ, 4β, 4aβ, 7β, 7aα, 8aβ)] 8a-[[1S,7R,9R]-2,5,8-Trioxa-3,7-dimethyl-cis-bicyclo[4.4.0]-dec-9-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 12 (200mg) in anhydrous tetrahydrofuran (10ml) was added, at room temperature under nitrogen atmosphere, solid mercuric trifluoroacetate (213mg) in small portions. The reaction mixture was allowed to stand at room temperature for 30 minutes and tributyltin hydride (135µl) was added with vigorous stirring. After 30 minutes ethyl acetate (50ml) was added and the suspension filtered through celite to give a colourless solution which was washed with water (3x100ml) and dried over magnesium sulfate. Elimination of the solvent gave an oil which was flash chromatographed over silica gel eluting with acetone:hexane (1:15) to give the title compound (120mg) as a 1:1 mixture of epimers at C-3'.
δ (¹H, CDCl₃) includes: 4.73 (dd, 1H, H-9', J=3.0 and 6.3 Hz), 4.62 (dd, 1H, H-9', J=2.1 and 9.9 Hz).

### INTERMEDIATE 15

### [1R-(1α, 3aβ, 4β, 4aβ, 7β, 7aα, 8aβ)] 8a-[[1S,7R,9S]-2,5,8-Trioxa-3,3,7-trimethyl-cis-bicyclo[4.4.0]-dec-9-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 13 (325mg) in anhydrous tetrahydrofuran (10ml) was added, at room temperature under nitrogen atmosphere, solid mercuric trifluoroacetate (303mg) in small portions. The reaction mixture was allowed to stand at room temperature for 30 minutes and tributyltin hydride (192µl) was added with vigorous stirring. After 30 minutes ethyl acetate (50ml) was added and the suspension filtered through celite to give a colourless solution which was washed with water (3x100ml) and dried over magnesium sulfate. Elimination of the solvent gave an oil which was flash chromatographed over silica gel eluting with acetone:hexane (1:20) and (1:15) to give the title compound (210mg) as a colourless oil.
δ (¹H, CDCl₃): 9.73 (s, 1H, CHO), 7.50-7.20 (m, 10H, 2Ph), 6.98 (s, 1H, CHPh₂), 6.03 (dd, 1H, H-2, J=1.2 Hz, J=3.3 Hz), 4.54 (dd, 1H, H-9', J=2.1 and 9.6 Hz), 4.25 (m, 2H, H-7' and H-1'), 4.05 and 3.66 (2d, 2H, 8aCH₂, J_{AB}=9 Hz), 3.41 and 3.18 (2d, 2H, OCH₂, J_{AB}=11.7 Hz), 3.25 (dd, 1H, H-6', J=3.3 and 10.2 Hz), 2.75 (t, 1H, H-1, J=3.3 Hz), 1.25 and 1.16 (2s, 2CH₃).

### INTERMEDIATE 16

### [1R-(1α, 3aβ, 4β, 4aβ, 7β, 7aα, 8aβ)] 8a-[2,6-Dideoxy-4-O-p-methoxybenzyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A suspension of Intermediate 10 (500mg) and tributyltin oxide (374mg) in dry toluene (50ml) was heated to reflux in a flask fitted with a Dean Stark condenser and then allowed to stand at room temperature under nitrogen atmosphere (approximately 10ml of azeotropic mixture were removed). p-Methoxybenzyl chloride (135µl) and tetrabutylammonium fluoride (1M solution in tetrahydrofuran, 1.5ml) were added consecutively and the mixture heated at 60^{o}C for 24 hours. Elimination of the solvent gave a residue which was flash chromatographed over silica gel eluting with acetone:hexane (1:20) and (1:15) to give the title compound (300mg) as a colourless oil.
δ (¹H, CDCl₃): 9.72 (s, 1H, CHO), 7.5-7.2 (m, 12H, 2Ph and 2Hortho), 6.97 (s, H, CHPh₂), 6.88 (m, 2H, 2Hmeta), 6.04 (dd, 1H, H-2, J=1.5 Hz, J=3.6 Hz), 4.64 (dd, 1H, H-1', J=2.4 and 9.9 Hz), 4.55 and 4.50 (2d, 2H, CH₂Ph, J_{AB}=11.2 Hz), 4.18 (m, 1H, H-3'), 4.04 and 3.66 (2d, 2H, 8aCH₂, J_{AB}=9 Hz), 3.80 (s, 3H, CH₃O), 3.74 (m, 1H, H-5'), 3.12 (dd, 1H, H-4', J=3.0 and 9.3 Hz), 2.75 (t, 1H, H-1, J=3.9 Hz).

### INTERMEDIATE 17

### [1R-(1α, 3aβ, 4β, 4aβ, 7β, 7aα, 8aβ)] 8a-[(3-O-Allyl-2,6-dideoxy-4-O-p-methoxybenzyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a vigorous stirred solution of Intermediate 16 (400mg) in anhydrous tetrahydrofuran (10ml) was added under nitrogen atmosphere at 0^{o}C sodium hydride (48mg) in small portions. After the addition was concluded the mixture was allowed to stand at room temperature for 1 hour and allyl bromide (191µl) was then added. After 24 hours the reaction was quenched by adding ammonium chloride (1N solution in water, 100ml) and the mixture was extracted with ethyl acetate (200ml). The organic layer was washed with water (1x100ml), dried over magnesium sulfate and concentrated to dryness to give an oil which was flash chromatographed over silica gel eluting with ethyl acetate:hexane (1:20) and (1:15) to give the title compound (400mg) as a colourless oil.
δ (¹H, CDCl₃): 9.74 (s, 1H, CHO), 7.5-7.2 (m, 12H, 2Ph and 2Hortho), 6.98 (s, H, CHPh₂), 6.86 (m, 2H, 2Hmeta), 6.04 (dd, 1H, H2, J=1.2 and 3.3 Hz), 5.95 (m, 1H, CH=CH₂), 5.28 and 5.15 (2m, 2H, CH=CH₂), 4.60 (m, 2H, H-1' and CH₂Ph), 4.42 (d, 1H, CH₂Ph, J_{AB}=11.4 Hz), 4.13 (m, 2H CH₂-CH=), 4.02 and 3.68 (2d, 2H, 8aCH₂, J_{AB}=9.6 Hz), 3.89 (m, 2H, H-5' and H-3'), 3.80 (s, 3H, CH₃O), 3.09 (dd, 1H, H-4', J=3.0 and 9.3 Hz), 2.73 (t, 1H, H-1, J=3.9 Hz).

### INTERMEDIATE 18

### [1R-(1α, 3aβ, 4β, 4aβ, 7β, 7aα, 8aβ)] 8a-[(3-O-Allyl-2,6-dideoxy-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 17 (2.3g) in dichloromethane (200ml) was stirred with water (20ml) and 2,3-dichloro-5,6-dicyanoquinone (0.79g) was added. After overnight stirring at room temperature the mixture was filtered through a Celite pad with the aid of more dichloromethane. The dichloromethane phase was then washed with aqueous sodium bicarbonate followed by sodium chloride solution, then dried over anhydrous sodium sulfate, filtered and concentrated to dryness. The residue was chromatographed on silica gel using dichloromethane: methanol (49:1) as the eluent to yield the title compound (1.95g) as a white foam.
δ (¹H, CDCl₃): 9.75 (s, 1H, CHO), 7.45-7.24 (m, 10H, Ph₂), 6.99 (s, 1H, CHPh₂), 6.05 (dd, 1H, H-2, J=1.5 and 3.3 Hz), 5.95 (m, 1H, CH=CH₂), 5.26 (m, 2H, CH=CH₂), 4.53 (dd, 1H, H-1', J=1.8 and 9.6 Hz), 4.19 and 3.96 (2m, 2H, CH₂CH=CH₂), 4.03 and 3.69 (2d, 2H, 8a-CH₂, J=9.3 Hz), 3.80 (q, 1H, H-3', J=3.3 Hz), 3.60 (dq, 1H, H-5', J=9.6 and 6.3 Hz), 3.23 (ddd, 1H, H-4', J= 10.8, 9.6 and 3.3 Hz), 2.74 (t, 1H, H-1, J=3.6 Hz), 2.25 (d, 1H, OH, J=10.8 Hz).

### INTERMEDIATE 19

### [1R-(1α, 3aβ, 4β, 4aβ, 7β, 7aα, 8aβ)] 8a-[(3-O-Allyl-2,6-dideoxy-4-O-(methylthio)thiocarbonyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a cooled solution of Intermediate 18 (1.9g) in anhydrous tetrahydrofuran (50ml) under nitrogen atmosphere were added sodium hydride in small portions (240mg) and a catalytic amount of imidazole. The ice bath was then removed and the reaction mixture was allowed to stand at room temperature under stirring for 1 hour. Carbon disulfide (1.5ml) and methyl iodide (1.25ml) were added consecutively. Once the reaction was completed (TCL control), the crude mixture was poured into ethyl acetate (500ml) and quenched with 0.1 N aqueous ammonium chloride (250ml). The organic layer was then washed with brine (1x500ml) and water (1x500ml), dried over anhydrous sodium sulfate and concentrated to dryness. The oily residue was flash chromatographed over silica gel using hexane:dichloromethane (3:5) as the eluent to obtain the title compound (1.85g) as a white foam.
δ (¹H CDCl₃): 9.74 (s, 1H, CHO), 7.45-7.24 (m, 10H, Ph₂), 6.99 (s, 1H, CHPh₂), 6.06 (dd, 1H, H-2, J=1.2 and 3.3 Hz), 5.88 (m, 1H, CH=CH₂), 5.41 (dd, 1H, H-4', J=2.7 and 9.6 Hz), 5.22 (m, 2H, CH=CH₂), 4.70 (dd, 1H, H-1', J=2.1 and 9.3 Hz), 4.20-4.02 (m, 5H, H-5', CH₂-CH=CH₂, H-3' and 8a-CH₂(1H)),3.71(d,1H, 8a-CH₂, J=9.3 Hz), 2.75 (t, 1H, H-1, J=3.9 Hz), 2.58 (s, 3H, SCH₃).

### INTERMEDIATE 20

### (a) [1R-(1α, 3aβ, 4β, 4aβ, 7β, 7aα, 8aβ)] 8a-[[1S,4S,6R,8R]-2,7-Dioxa-4,6-dimethyl-cis-bicyclo[3.4,0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester and

### (b) [1R-(1α, 3aβ, 4β, 4aβ, 7β, 7aα, 8aβ)] 8a-[[1S,4R,6R,8R]-2,7-Dioxa-4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 19 (1.8g) in dry toluene (250ml) was degassed with an argon stream for 1 hour and then heated to reflux. A catalytic amount of azobis(isobutyronitrile) and tributyltin hydride (0.95ml) dissolved in dry toluene (50ml) were added from a syringe in about 2 hours while heating and the argon stream were maintained. Once the addition was completed the reaction mixture was further refluxed for 1 hour and subsequently cooled, then concentrated to dryness and flash chromatographed on silica gel using hexane and then hexane:ethyl acetate (9:1) as the eluents. Intermediate 20(a) (680mg; Rf=0.31 in hexane:ethyl acetate 4:1) was obtained as a white foam and Intermediate 20(b) (180mg; Rf = 0.25 in hexane:ethyl acetate 4:1) was isolated as a white solid.
(a) δ (¹H, CDCl₃): 9.73 (s, 1H, CHO), 7.45-7.28 (m, 10H, Ph₂), 6.99 (s, 1H, CHPh₂), 6.05 (dd, 1H, H-2, J=1.2 and 3.6 Hz), 4.46 (dd, 1H, H-8', J=2.4 and 9.6 Hz), 4.14-4.05 (m, 3H, 8a-CH₂ (1H), H-1' and CH₂-3' (1H)), 3.67 (d, 1H, 8a-CH₂, J=9.3 Hz), 3.36-3.25 (m, 2H, H-6' and CH₂-3' (1H)), 2.77 (t, 1H, H-1, J=3.9 Hz), 1.05 (d, 3H, 4'-CH₃, J=6.9 Hz).
(b) δ (¹H, CDCl₃): 9.72 (s, 1H, CHO), 7.45-7.24 (m, 10H, Ph₂), 6.99 (s, 1H, CHPh₂), 6.05 (dd, 1H, H-2, J=1.50 and 3.6 Hz), 4.42 (dd, 1H, H-8', J=2.1 and 9.6 Hz), 4.20 (m, 1H, H-1'), 4.07 and 3.67 (2d, 2H, 8a-CH₂, J=9.3 Hz), 3.97 (t, 1H, CH₂-3', J=8.4 Hz),3.54 (m, 1H, H-6'), 3.46 (dd, 1H, CH₂-3', J=10.5 Hz), 2.77 (t, 1H, H-1, J=3.9 Hz), 2.53 (m, 1H, H-4'), 1.02 (d, 3H, 6-CH₃).

### INTERMEDIATE 21

### [1R-(1α,3aβ,4β,4aβ,7β,7aα,8aβ)] 8a-[(4-O-Allyl-2,6-dideoxy-3-O-(methylthio)thiocarbonyl-β-D-allopyranosyloxy)methyl]-4-formyl-4-4a,5,6,7,7a,8,8a-octahydro-7-methyl-3-(1-methylethyl)-1,4-methano-s-indacene-3a(1H)-carboxylic acid, diphenylmethyl ester

To a solution of Intermediate 12 (380mg) in anhydrous tetrahydrofuran (10ml) were added, under nitrogen atmosphere at 0^{o}C, solid sodium hydride (48mg) in small portions and a catalytic amount of imidazole. Once the addition was concluded, the cool bath was removed and the reaction was allowed to stand at room temperature for 1 hour. Carbon disulfide (300µl) and methyl iodide (250µl) were added consecutively. After 4 hours the mixture was poured into ethyl acetate (100ml) and quenched with aqueous 1N ammonium chloride (50ml). The organic layer was washed with brine (1x100ml) and water (1x100ml), dried over magnesium sulfate and concentrated to dryness to give a yellow oil which was flash chromatographed over silica gel eluting with ethyl acetate:hexane (1:25) and (1:20) to afford the title compound (350mg) as a white foam.
δ (¹H, CDCl₃): 9.73 (s, 1H, CHO), 7.50-7.20 (m, 10H, 2Ph), 6.98 (s, H, CHPh₂), 6.24 (m, 1H, H-3'), 6.05 (dd, 1H, H-2, J=1.5 and 3.6 Hz), 5.85 (m, 1H, CH=CH₂), 5.30-5.20 (m, 2H, CH=CH₂), 4.56 (dd, 1H, H-1', J=1.8 and 9.3 Hz), 4.15-3.75 (m, 4H, CH₂CH=CH₂+8aCH₂+H-5'), 3.69 (d, 1H, 8aCH₂, J=9.3 Hz), 3.21 (dd, 1H, H-4', J=3.3 and 9.3 Hz), 2.72 (t, 1H, H-1, J=3.89 Hz), 2.59 (s, 3H, SCH₃).

### INTERMEDIATE 22

### [1R-(1α, 3aβ, 4β, 4aβ, 7β, 7aα, 8aβ)] 8a-[(2,6-Dideoxy-3-O-(methylthio)thiocarbonyl-4-O-(2-methyl-2-propenyl)-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

To a cooled solution of Intermediate 13 (700mg) in anhydrous tetrahydrofuran (30ml) under nitrogen atmosphere were added sodium hydride (85mg) in small portions and a catalytic amount of imidazole. The ice bath was then removed and the reaction mixture was allowed to stand at room temperature under magnetical stirring for 1 hour. Carbon disulfide (0.54ml) and methyl iodide (0.45ml) were added consecutively. Once the reaction was completed (TLC control), the crude mixture was poured into ethyl acetate (200ml) and quenched with 0.1 N aqueous ammonium chloride (120ml). The organic layer was then washed with brine (1 x 200ml) and water (1 x 200ml), dried over anhydrous sodium sulfate and concentrated to dryness. The oily residue thus obtained was chromatographed on silica gel using hexane:ethyl acetate (19:1) as the eluent to give the title compound (600mg).
δ(¹H, CDCl₃): 9.73 (s, 1H, CHO),7.45-7.26 (m, 10H, Ph₂), 6.98 (s, 1H, CHPh₂), 6.24 (m, 1H, H-3'), 6.05 (dd, 1H, H-2, J=1.5 and 3.6 Hz), 4.92 and 4.88 (2m, 2H, C=CH₂), 4.55 (dd, 1H, H-1', J=2.1 and 9.6 Hz), 4.03 and 3.69 (2d, 2H, 8a-CH₂, J=9.3 Hz), 3.95 and 3.82 (2d, 2H, CH₂-C=CH₂, J=12.3 Hz), 3.86 (m, 1H, H-5'), 3.19 (dd,1H, H-4', J=3 and 9.3 Hz), 2.72 (t, 1H, H-1, J=3.9 Hz), 2.58 (s, 3H, SCH₃), 1.70 (s, 3H, CH₃C=CH₂).

### INTERMEDIATE 23

### (a) [1R-(1α,3aβ,4β,4aβ,7β,7aα,8aβ)] 8a-[[1S,4R,7R,9R]-2,8-Dioxa-4,9-dimethyl-cis-bicyclo[3.4.0]-non-7-yl-oxy-methyl]-4-formyl-4-4a,5,6,7,7a,8,8a-octahydro-7-methyl-3-(1-methylethyl)-1,4-methano-s-indacene-3a(1H)-carboxylic acid, diphenylmethyl ester and

### (b) [1R-(1α, 3aβ, 4β, 4aβ, 7β, 7aα, 8aβ)] 8a-[[1S,4S,7R,9R]-2,8-Dioxa-4,9-dimethyl-cis-bicyclo[3.4.0]-non-7-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 21 (1.5g) in dry toluene (150ml) was degassed with argon for 90 minutes and then heated to reflux. To this boiling solution were added tributyltin hydride (847µl) and azobis(isobutyronitrile) (33mg). Heating was continued for an additional period of 30 minutes. Elimination of solvent gave a crude mixture which was flash chromatographed twice on silica gel eluting with hexane:ethyl acetate (20:1) to give title compound (a) (150mg; Rf=0.6 in hexane:ethyl acetate 4:1) and title compound (b) (260mg; Rf=0.4 in hexane:ethyl acetate 4:1) both as colourless oils.
(a) δ (¹H, CDCl₃): 9.75 (s, 1H, CHO), 7.5-7.2 (m, 10H, 2Ph), 6.99 (s, 1H, CHPh₂), 6.06 (dd, 1H, H-2, J=1.5 and 3.6 Hz), 4.46 (dd, 1H, H-7', J=3 and 7.5 Hz), 4.06-3.95 (m, 2H, H-3' and 8aCH₂), 3.78-3.60 (m, 2H, H-1' and 8aCH₂), 3.38-3.26 (m, 2H, H-9' and H-3'), 2.75 (t, 1H, H-1, J=3.6 Hz), 0.98 (d, 3H, CH₃,CH, J=6.9 Hz).
(b) δ (¹H, CDCl₃): 9.74 (s, 1H, CHO), 7.5-7.2 (m, 10H, 2Ph), 6.99 (S, 1H, CHPh₂), 6.04 (dd, 1H, H-1, J=1.5 and 3.6 Hz), 4.74 (t, 1H, H-7', J=3 Hz), 3.94 and 3.61 (2d, 2H, 8aCH₂, J_{AB}=9.3 Hz), 3.88 and 3.76 (2d, 2H, H-3'), 3.66 (m, 1H, H-9'), 3.43 (t, 1H, H-1', J=7.8 Hz), 2.72 (t, 1H, H-1, J=3.6 Hz), 2.46 (2m, 2H, CHCH₂O and H-5'), 0.97 (d, 3H, CH₃CH, J=6.9 Hz).

### INTERMEDIATE 24

### [1R-(1α, 3aβ, 4β, 4aβ, 7β, 7aα, 8aβ)] 8a-[[1S,7R,9R]-2,8-Dioxa-4,4,8-trimethyl-cis-bicyclo[3.4.0]-non-7-yl-oxy-methyl]-4-formyl-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 22 (580mg) in dry toluene (150ml) was degassed with an argon stream for one hour and then heated to reflux. A catalytic amount of azobis(isobutyronitrile) and tributyltin hydride (0.27ml) dissolved in dry toluene (20ml) were added from a syringe over about 2 hours while heating and the argon stream were maintained. Once the addition was completed, the reaction mixture was further refluxed for 3 hours and subsequently cooled, then concentrated and chromatographed on silica gel using hexane and then hexane:ethyl acetate (4:1) as eluents to give the title compound (100mg) as a white foam.
δ(¹H, CDCl₃): 9.74 (s, 1H CHO), 7.45-7.26 (m, 10H, Ph₂), 6.99 (s, 1H, CHPh₂), 6.04 (dd, 1H, H-2, J=1.5 and 3.6 Hz), 4.71 (t, 1H, H-7' J=3.3 Hz), 3.93 and 3.61 (2d, 2H, 8a-CH₂, J=9.3 Hz), 3.85 (t, 1H, H-1', J=8.7 Hz), 3.56 (m, 1H, H-9'), 3.55 and 3.43 (2d, 2H, CH₂-3', J=8.4 Hz), 2.73 (m, 1H, H-1), 1.08 and 0.99 (2s, 6H, 4'-CH₃).

### INTERMEDIATE 25

### [1R-(1α, 3aβ, 4β, 4aβ, 7β, 7aα, 8aβ)] 8a-[(6-Deoxy-β-D-altropyranosyloxy) methyl]-4-(1,3-dioxolan-2-yl)-4,4a,5,6,7,7a,8,8a-octahydro-7-methyl-3-(1-methylethyl)-1,4-methano-s-indacene-3a(1H)-carboxylic acid, diphenylmethyl ester

To a stirred solution of Intermediate 1 (2.5g) in dry acetonitrile (100ml) were added at room temperature ethylene glycol (30ml), trimethyl orthoformate (2ml) and a catalytic amount of *p*-toluensulfonic acid. The reaction mixture was stirred for 3 hours, then diluted with ethyl acetate (300ml) and washed sucessively with 5% aqueous sodium bicarbonate and brine. The organic layer was dried over anhydrous sodium sulfate, concentrated and purified by silica gel chromatography using hexane:ethyl acetate (3:1) as the eluent to give the title compound (2.6g).
δ (¹H CDCl₃): 7.46-7.24 (m, 10H, Ph₂), 6.94 (s, 1H, CHPh₂), 5.83 (dd, 1H, H-2, J=1.2 and 3.6 Hz), 5.07 (s, 1H, CH beta), 4.65 (d, 1H, H-1', J=1.8 Hz), 4.07-4.04 (m, 2H, 8a-CH₂ (1H) and H-3'), 3.88-3.69 (m, 8H, OCH₂CH₂O,8a-CH₂(1H), H-2', H-4' and H-5'), 2.51 (t, 1H, H-1, J=4.5 Hz), 2.37 (d, 1H, OH, J=2.7 Hz), 2.28 (d, 1H, OH, J=3.6 Hz), 2.04 (d, 1H, OH, J=5.7 Hz).

### INTERMEDIATE 26

### [1R-(1α, 3aβ, 4β, 4aβ, 7β, 7aα, 8aβ)] 8a-[(6-Deoxy-3,4-O-isopropylidene-β-D-altropyranosyloxy)methyl]-4-(1,3-dioxolan-2-yl)-4,4a,5,6,7,7a,8,8a-octahydro-7-methyl-3-(1-methylethyl)-1,4-methano-s-indacene-3a(1H)-carboxylic acid, diphenylmethyl ester

A solution of Intermediate 25 (200mg) in dry acetonitrile (6ml) under nitrogen was treated with 2,2-dimethoxypropane (0.6ml) and a catalytic amount of pyridinium *p*-toluenesulfonate. After 4 hours at room temperature the mixture was treated with saturated aqueous sodium hydrogen carbonate solution (20ml) and extracted twice with ethyl acetate (20ml). The combined organic phases were washed with water and brine, dried over magnesium sulphate, filtered and evaporated. The residue was purified by flash chromatography on silica gel eluting with hexane:ethyl acetate (4:1) and appropriate fractions were combined and evaporated to give the title compound (205mg) as a white foam.
δ (¹H, CDCl₃): 7.47-7.2 (m, 10H,2Ph), 6.93 (s, 1H, CHPh₂), 5.83 (dd, 1H, H-2, J=1.2 and 3.6 Hz), 5.08 (s, 1H, 1,3-dioxolane), 4.56 (d, 1H, H-1', J=2.1 Hz), 4.3 (dd, 1H, H-3', J=3.6 and 5.7 Hz), 4.04 (d, 1H, A part of 8aCH₂, J_{AB}=9 Hz), 3.95-3.75 (m, 7H, H-2', H-4', OCH₂CH₂O and B part of 8aCH₂), 3.44 (dq, 1H, H-5', J=6 and 9 Hz), 2.65 (m, 1H, CHMe₂), 2.51 (bt, 1H, H-1, J=3.6 Hz), 2.35 (d, 1H, OH2'), 1.47, 1.36 (2s, 6H, 2CH₃ isopropylidene ketal).

### INTERMEDIATE 27

### [1R-(1α, 3aβ, 4β, 4aβ, 7β, 7aα, 8aβ)] 8a-[(6-Deoxy-3,4-O-isopropylidene-β-D-allopyranosyloxy) methyl]-4-(1,3-dioxolan-2-yl)-4,4a,5,6,7,7a,8,8a-octahydro-7-methyl-3-(1-methylethyl)-1,4-methano-s-indacene-3a(1H)-carboxyllic acid, diphenylmethyl ester

Pyridine (0.67ml) was added to a mixture of chromium oxide (CrO₃) (412mg) in dichloromethane (25ml) at 0^{o}C. After 15 minutes, acetic anhydride (0.40ml) was added followed after 10 minutes at the same temperature by a solution of Intermediate 26 (750mg) in dichloromethane (25ml). The mixture was stirred for 2 hours, filtered through a silica gel column with a top layer of magnesium sulfate and the filtrate was evaporated to dryness under reduced pressure to afford a syrup. This was dissolved in methanol:water (10:1; 11ml), and then sodium borohydride (40mg) was added at 0^{o}C. The mixture was kept at 0°C for 2 hours, acidified to pH 6-7 with a solution of 1N hydrochloric acid, and then evaporated to dryness under reduced pressure to afford a white solid, which was purified twice by flash column chromatography on silica gel eluting successively with hexane:ethyl acetate (6:1) then (2:1). The fractions which contained the desired product were combined and evaporated in vacuo to yield the title compound (585mg).
δ (¹H, CDCl₃): 7.24-7.46 (m, 10H, 2Ph), 6.92 (s, 1H, CHPh₂), 5.87 (dd, 1H, H-2, J= 3.9 and 1.5 Hz), 5.09 (s, 1H, CH-dioxolane), 4.52 (t, 1H, H-3', J= 4.5 Hz), 4.45 (d, 1H, H-1', J= 7.8 Hz, 4.07 (d, 1H, 8aCH₂, J= 9 Hz), 3.74-3.86 (m, 6H, 2CH₂-dioxolane, H-4', 8aCH₂), 3.67 (m, 1H, H-2'), 2.65 (m, 1H, H-14), 2.58 (t, 1H, H-11).

### INTERMEDIATE 28

### [1R-(1α, 3aβ, 4β, 4aβ, 7β, 7aα, 8aβ)] 4-Formyl-4,4a,5,6,7,7a,8,8a-octahydro-8a-hydroxymethyl-7-methyl-3-(1-methylethyl)-1,4-methano-s-indacene-3a(1H)-carboxylic acid, 2-(trimethylsilyl)ethyl ester

A solution of sordaricin (6.6g) and *O*-[2-(trimethylsilyl)ethyl]-N,N'-diisopropylisourea¹ (9.3g) in anhydrous tetrahydrofuran (150ml) was heated under reflux for 3 hours. The mixture was then cooled to room temperature, filtered and concentrated to dryness. The residue was dissolved in ethyl acetate (750ml) and washed successively with 1N hydrochloric acid, 5% aqueous sodium bicarbonate and brine. The organic layer was dried over anhydrous sodium sulfate, concentrated in vacuo and purified by silica gel flash column chromatography using (10%) ethyl acetate in hexane as eluent to give the title compound (6.8g) as a white foam.
δ (¹H, CDCl₃): 9.67 (s, 1H, CHO), 6.07 (dd, 1H, H-2, J=1.5 and 3.6 Hz), 4.27 (m, 2H, CH₂CH₂O), 3.93 and 3.49 (2m, 2H, 8a-CH₂), 2.75 (bs,1H, OH), 2.54 (t, 1H, H-1), 1.05 (m, 2H, CH₂CH₂Si), 0.07 (s, 9H, (CH₃)₃Si); δ (¹³C, CDCl₃): 204.9 (CHO), 173.6 (COOTMSE), 148.2 (C-3), 130.6 (C-2), 66.9 (8a-CH₂O), 64.0 (CH₂CH₂O), 177.7 (CH₂CH₂Si), 1.6 (CH₃)₃Si).
¹ L.J. Mathias, Synthesis, (1979), 561.

### INTERMEDIATE 29

### [1R-(1α, 3aβ, 4β, 4aβ, 7β, 7aα, 8aβ)] 8a-[(3,4,6-Tri-O-acetyl-2-deoxy-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, trimethylsilylethyl ester

To a solution of Intermediate 28 (63mg) and 3,4,6-tri-*O*-acetyl D-allal¹ (20mg) in dry toluene (1ml) was added hydrobromic acid-triphenyl phosphine (9mg). The reaction mixture was heated at 60^{o}C for 2 hours and poured into a 1M solution of sodium bicarbonate (100ml). Ethyl acetate (100ml) was added and the organic layer was washed with water, dried over anhydrous magnesium sulfate and filtered. The solvent was evaporated to dryness, and the residue was purified by silica gel flash column chromatography eluting with hexane:ethyl acetate (10:1). to give the title compound (16mg) as a white foam.
δ (¹H, CDCl₃): 9.73 (s, 1H, CHO), 6.03 (dd, 1H, H-2, J=1.5 and 3.6 Hz), 5.48 (m, 1H, H-3'), 4.89 (dd, 1H, H-4', J=3.3 and 9.6 Hz), 4.65 (dd, 1H, H-1', J=2.4 and 8.7 Hz), 4.23 (m, 4H, H-6', COOCH₂CH₂ Si), 4.02 (ddd, 1H, H-5', J= 3, 5.4 and 9.6 Hz), 3.92 and 3.69 (2d, 2H, 8aCH₂, J=9.6 Hz), 2.75 (t, 1H, H-1), 2.28 (m, 1H, CH(CH₃)2).
1. M. D. Wittman, R. L. Halcomb and S. J. Danishefsky, J. Org. Chem. (1990), 55, 1979-1981.

### INTERMEDIATE 30

### [1R-(1α, 3aβ, 4β, 4aβ, 7β, 7aα, 8aβ)] 8a-[(2-Deoxy-3,4-O-isopropylidene-6-O-methyl-β-D-allopyranosyioxy)methyl]-4-formyl-4,4a,5,6,7,7a,8,8a-octahydro-7-methyl-3-(1-methylethyl)-1,4-methano-s-indacene-3a(1H)-carboxylic acid,2-(trimethylsilyl)ethyl ester

To a stirred solution of Intermediate 29 (275mg) in absolute methanol (10ml) was added 2 or 3 drops of 1 M solution of sodium methoxide in methanol. After stirring for 1.5 hours the reaction mixture was concentrated, diluted with toluene (10ml) and concentrated to leave a yellow syrup. This was dissolved in acetone (5ml) and 2,2-dimethoxypropane (60µl) and p-toluensulfonic acid monohydrate (75mg) were added. The reaction mixture was stirred at room temperature for 1 hour, neutralized with sodium carbonate, filtered and the filtrate concentrated to dryness. The oily residue was dissolved in ethyl acetate (20ml), washed with water and brine, dried and concentrated to give an oil. A solution of this oil in dry tetrahydrofuran (5ml) at 0^{o}C was treated with sodium hydride (10mg) and stirred for 30 minutes before methyl iodide (100µl) was added. The reaction mixture was gradually warmed to room temperature during 3 hours. The reaction was quenched with methanol (1ml) and the mixture was then concentrated, the residue purified three times by flash column chromatography using successively hexane:ethyl acetate (10:1), (6:1) and (4:1) and the appropiate fractions were combined and the solvents evaporated to give the title compound (50mg) as a colourless oil.
δ (¹H, CDCl₃): 9.73 (s, 1H, CHO), 6.04 (dd, 1H, H-2, J)1.2 and 3.6 Hz), 4.61 (dd, 1H, H-1', J=2.4 and 8.4 Hz), 4.43 (m, 1H, H-3'), 4.34-4.10 (m, 2H, CH₂-SEM protecting group), 3.66 and 3.89 (2d, 2H, 8aCH₂, J=9.3 Hz), 3.87 (m, 1H, H-4'), 3.48-3.64 (m, 3H, 2H-6' and H-5'), 3.24 (s, 3H,6'OMe), 2.73 (t, 1H, H-1, J=3.6 Hz), 1.34 and 1.35 (2s, 6H, methyl groups of isopropylidene).

### EXAMPLE 1

### [1R-(1α, 3aβ, 4β, 4aβ, 7β, 7aα, 8aβ)] 8a-[(6-Deoxy-3,4-O-isopropylidene-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (80mg) in ethyl acetate (10ml) under nitrogen was added a solution of Intermediate 2 (180mg) in ethyl acetate (10ml) and the mixture was hydrogenated at room temperature under 30 psi of hydrogen for 1 hour. The catalyst was filtered off and the solvent evaporated to dryness. Flash chromatography of the residue on silica gel eluting with dichloromethane:methanol (20:1) gave the title compound (128mg).
δ(¹H, CDCl₃): 9.73 (s, 1H, CHO), 6.08 (dd, 1H, H-2, J=1.5 and 3.3 Hz), 4.61 (d, 1H, H-1', J=2.1 Hz), 4.30 (dd, 1H, H-3', J=3.6 and 5.4 Hz), 4.04 (d, 1H, 8aCH₂, J=9.3 Hz), 3.95 (dd, 1H, H-2', J=2.1 and 3.6 Hz), 3.85 (dd, 1H, H-4', J=5.4 and 9.0 Hz), 3.66 (d, 1H, J=9.3 Hz), 3.49 (dq, 1H, H-5', J_{d}=9.0 Hz, J_{q}=6.3 Hz), 2.69 (t, 1H, H-1, J=3.6 Hz); δ (¹³C, CDCl₃): 204.8 (CHO), 175.4 (CO₂H), 148.3 (C-3), 130.7 (C-2), 109.3 (Cq.isop), (98.9 (C-1'), 76.9 (C-4'), 75.8 (C-2'), 74.2 (8aCH₂), 71.7 (C-3'), 68.6 (C-5'), 65.6 (C-8a), 58.9 (C-4).

### EXAMPLE 2

### [1R-(1α, 3aβ, 4β, 4aβ, 7β, 7aα, 8aβ)] 8a-[(6-Deoxy-3,4-O-isopropylidene-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid, sodium salt

Example 1 (246mg) was dissolved in dioxane (2ml) and 0.5 M sodium bicarbonate (0.99ml) was added. The solution was stirred for 15 minutes and freeze-dried to obtain the title compound (256mg).
δ (¹H, CDCl₃): 9.88 (s, 1H, CHO), 5.95 (dd, 1H, H-2, J=1.5 and 3.3 Hz), 4.51 (d, 1H, H-1', J=1.8 Hz), 4.26 (dd, 1H, H-3', J=4.8 and 6.0 Hz), 4.01 (d, 1H, 8aCH₂, J=9.6 Hz), 3.87-3.80 (m, 2H, H-2' and H-4'), 3.74 (d, 1H, 8aCH₂, J=9.6 Hz), 3.50-3.42 (m, 1H, H-5'), 2.66 (t, 1H, H-1, J=3.9 Hz).

### EXAMPLE 3

### [1R-(1α, 3aβ, 4β, 4aβ, 7β, 7aα, 8aβ)] 8a-[(6-Deoxy-3,4-O-(2-pentylidene)-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (30mg) in ethyl acetate (10ml) under nitrogen, was added a solution of Intermediate 3 (140mg) in ethyl acetate and the mixture was hydrogenated under 30 psi of hydrogen for 45 minutes. The catalyst was filtered off and the solvent evaporated to dryness. Flash chromatography on silica gel using dichloromethane:methanol (30:1) as eluent afforded the title compound (75mg) in a epimer ratio of 26:74.
δ (¹H, CDCl₃): 9.82 (s, 1H, CHO), 6.08 (dd, 1H, H-2, J=1.5 and 3.3 Hz), 4.60 (d, 1H, H-1', J=1.8 Hz), 4.30 (dd, 1H, H-3', J=4.2 and 6.0 Hz), 3.98 (d, 1H, 8aCH₂, J=9.3 Hz), 3.94 (dd, 1H, H-2', J=1.8 and 4.2 Hz), 3.87 (dd, 1H, H-4', J=6.0 and 9.3 Hz), 3.65 (d, 1H, 8aCH₂, J=9.3 Hz), 3.47 (dq, 1H, H-5', J_{d}=9.3 Hz, J_{q}=6.3 Hz), 2.70 (t, 1H, H-1, J=3.9 Hz); δ (¹³C, CDCl₃): 204.5 (CHO), 176.5 (CO₂H), 148.2 (C-3), 130.6 (C-2), 110.8 (Cq acetal), 98.8 (C-1'), 76.6 (C-4'), 75.6 (C-2'),73.9 (8aCH₂), 71.3 (C-3'), 68.9 (C-5'), 65.5 (C8a), 58.8 (C-4).

### EXAMPLE 4

### [1R-(1α, 3aβ, 4β, 4aβ, 7β, 7aα, 8aβ)] 8a-[(6-Deoxy-3,4-O-(4-methoxy-2-butylidene)-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (100mg) in ethyl acetate (15ml) was added a solution of Intermediate 4 (260mg) in ethyl acetate (10ml) and the mixture was hydrogenated at room temperature under 30 psi of hydrogen for 45 minutes. The catalyst was filtered off and the solvent evaporated to dryness. The residue was flash chromatographed on silica gel eluting with dichloromethane:methanol (30:1) to give the title compound (172mg).
δ (¹H, CDCl₃): 9.71 (s, 1H, CHO), 6.08 (dd, 1H, H-2, J=1.5 and 3.6 Hz), 4.60 (d, 1H, H-1', J=2.4 Hz), 4.31 (dd, 1H, H-3', J=4.2 and 6.0 Hz), 3.98 (d, 1H, 8aCH₂, J=9.3 Hz), 3.94 (dd, 1H, H-2', J=2.4 and 4.2 Hz), 3.88 (dd, 1H, H-4', J=6.0 and 9.3 Hz), 3.65 (d, 1H, 8aCH₂, J=9.3 Hz), 3.50 (t, 1H, CH₂O, J=7.2 Hz), 3.50-3.40 (m, 1H, H-5'), 2.69 (t, 1H, H-1, J=3.9 Hz); δ (¹³C, CDCl₃): 204.5 (CHO), 176.2 (CO₂H), 148.2 (C-3), 130.6 (C-2), 109.8 (C-2''), 98.8 (C-1'), 76.7 (C-4'), 75.7 (C-2'), 73.8 (8aCH₂), 71.2 (C-2'), 68.8 (C-5'), 68.4 (CH₂O), 65.6 (C8a), 58.9 (C-4), 58.5 (CH₃O).

### EXAMPLE 5

### [1R-(1α, 3aβ, 4β, 4aβ, 7β, 7aα, 8aβ)] 8a-[(6-Deoxy-3,4-O-cyclopentylidene-β-D-altropyranosyloxy) methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (50mg) in ethyl acetate (10ml) was added a solution of Intermediate 5 (232mg) in ethyl acetate and the mixture was hydrogenated at room temperature under 35 psi of hydrogen for 1.5 hours. The catalyst was filtered off and the solvent evaporated to dryness. The residue was flash chromatographed on silica gel eluting with dichloromethane:methanol (30:1) to give the title compound (67mg).
δ (¹H, CDCl₃): 9.71 (s, 1H, CHO), 6.07 (dd, 1H, H-2, J=1.5 and 3.3 Hz), 4.59 (d, 1H, H-1', J=2.1 Hz), 4.17 (dd, 1H, H-3', J=3.6 and 5.4 Hz), 4.54 (d, 1H, 8aCH₂, J=9.3 Hz), 3.97 ( dd, 1H, H-2', J=2.1 and 3.6 Hz), 3.81 (dd, 1H, H-4', J=5.4 and 9.0 Hz), 3.70 (d, 1H, 8aCH₂, J=9.3 Hz), 3.44 (dq, 1H, H-5', J_{d}=9.0 Hz, J_{q}=6.3 Hz), 2.69 (t, 1H, H-1, J=3.3 Hz).

### EXAMPLE 6

### [1R-(1α, 3aβ, 4β, 4aβ, 7β, 7aα, 8aβ)] 8a-[(2,6-Dideoxy-3,4-O-isopropylidene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 7 (1.2g) in ethyl acetate (60ml) was added 10% palladium on charcoal (600mg) in portions, under nitrogen. The mixture was shaken in a Parr apparatus under 40 psi of hydrogen for 1 hour at room temperature. The catalyst was filtered off and washed with more ethyl acetate. The solvent was evaporated to dryness and the residue was flash chromatographed on silica gel eluting with dichloromethane:methanol (25:1) to give the title compound (0.79g).
δ (¹H, CDCl₃): 9.81 (s, 1H, CHO), 6.05 (dd, 1H, H-2, J=1.2 and 3.3 Hz), 4.63 (dd, 1H, H-1', J=2.4 and 8.4 Hz), 4.38 (dt, 1H, H-3', J_{d}=3.0 Hz, Jₜ=5.1 Hz), 4.29 (d, 1H, 8aCH₂, J=9.3 Hz), 3.64 (dd, 1H, H-4', J=5.1 and 9.3 Hz), 4.43 (dq, 1H, H-5', J_{d}=9.3 Hz, J_{q}=6.0 Hz), 3.40 (d, 1H, 8aCH₂, J=9.3 Hz), 2.53 (t, 1H, H-1, J=3.9 Hz); δ (¹³C, CDCl₃): 204.7 (CHO), 174.6 (CO₂H), 148.2 (C-3), 130.6 (C-2), 108.9 (C(CH₃)₂), 98.3 (C-1'), 76.6 (C-4'), 73.7 (8aCH₂), 72.5 (C-3'), 71.7 (C-5'), 67.0 (C8a), 65.2, 59.0 (C-4).

### EXAMPLE 7

### [1R-(1α, 3aβ, 4β, 4aβ, 7β, 7aα, 8aβ)] 8a-[(2,6-Dideoxy-3,4-O-(4-methoxy-2-butylidene)-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a suspension of 10% palladium on charcoal (300mg) in ethyl acetate (10ml) was added a solution of Intermediate 8 (246mg) in ethyl acetate (10ml) and the mixture was hydrogenated at room temperature under 45 psi of hydrogen for 1 hour. The catalyst was filtered off and the solvent evaporated to dryness. The residue was purified by flash chromatography on silica gel eluting with dichloromethane and dichloromethane:methanol (30:1) to give the title compound (182mg) as a white foam in an epimer ratio of 4:1.
δ (¹H, CDCl₃) signals of the major component: 9.81 (s, 1H, CHO), 6.06 (dd, 1H, H-2, J=1.5 and 3.6 Hz), 4.62 (dd, 1H, H-1', J=2.7 and 8.1 Hz), 4.41 (dt, 1H,H-3', J_{d}=3.3 Hz, Jₜ=2.5 Hz), 4.24 (d, 1H, 8aCH₂, J=9.0 Hz), 3.68 (dd, 1H, H-4', J=5.7 and 9.0 Hz), 3.50 (t, 2H, CH₂, J=7.5 Hz), 3.46-3.38 (m, 1H, H-5'), 3.33 (s, 3H, CH₃O), 2.54 (t, 1H, H-1, J=4.2 Hz),; δ (¹³C, CDCl₃): 204.5 (CHO), 175.1 (CO₂H), 148.2 (C-3), 130.7 (C-2), 109.4 (C-2''), 98.3 (C-1'), 76.4 (C-4'), 73.6 (8aCH₂), 71.9 (C-3'), 71.4 (C-5'), 68.6 (CH₂O), 65.2 (C8a), 58.9 (C-4), 58.6 (CH₃O).

### EXAMPLE 8

### [1R-(1α, 3aβ, 4β, 4aβ, 7β, 7aα, 8aβ)] 8a-[(2,6-Dideoxy-3,4-O-isopropylidene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, sodium salt

Example 6 (376mg) was dissolved in dioxane (3ml) and 0.5 N sodium bicarbonate (1.50ml) was added. The resulting solution was stirred for 30 minutes at room temperature and then freeze-dried to give the title compound (392mg).
δ (¹H, CDCl₃): 9.84 (s, 1H, CHO), 5.94 (dd, 1H, H-2, J=1.5 and 3.6 Hz), 4.52 (dd, 1H, H-1', J=2.4 and 9.0 Hz), 4.41-4.37 (m, 1H, H-3'), 4.02 (d, 1H, 8aCH₂, J=9.6 Hz), 3.74 (d, 1H, 8aCH₂, J=9.6 Hz), 3.57 (dd, 1H, H-4', J=5.4 and 10.0 Hz), 3.42 (dq, 1H, H-5', J_{d}=6.3 Hz, J_{q}=6.3 Hz), 2.56 (t, 1H, H-1, J=3.6 Hz).

### EXAMPLE 9

### [1R-(1α, 3aβ, 4β, 4aβ, 7β, 7aα, 8aβ)] 8a-[(6-Deoxy-3,4-O-thionocarbonyl-β-D-altropyranosyloxy)methyl]-4-formyl-4,4a,5,6,7,7a,8,8a-octahydro-7-methyl-3-(1-methylethyl)-1,4-methano-s-indacene-3a(1H)-carboxylic acid

A 2% solution of trifluoroacetic acid in dicloromethane (20ml) was added to a solution of Intermediate 9 (0.220g) in dichloromethane (5ml) at 0^{o}C. The reaction mixture was stirred at 0^{o}C for 1 hour and then washed with water (2x20ml) and dried (magnesium sulfate). The solvent was evaporated to give a residue which was chromatographed on silica gel column eluting with dichloromethane:methanol (40:1 to 20:1) to give the title compound (0.1g).
δ (¹H, CDCl₃): 9.68 (s, 1H, CHO), 6.09 (dd, 1H, H-2, J=1.5 and 3.6 Hz), 4.94 (dd, 1H, H-3', J=3.9 and 7.2 Hz), 4.62 (m, 2H, H-1' and H-4'), 4.14 (dd, 1H, H-2', J=2.1 and 3.9 Hz), 4.00 (d, 1H, 8aCH₂, J_{AB}=9.3 Hz), 3.72 (d, 1H, 8aCH₂, J_{AB}=9.3 Hz), 3.68 (m, 1H, H-5'), 2.71 (m, 1H, H-1); δ (¹³C, CDCl₃): 204.4 (CHO), 190.2 (CS), 176.4 (CO₂H), 148.4 (C-3), 130.6 (C-2), 98.2 (C-1'), 82.3 and 80.1 (C-3' and C-4'), 74.5 (8aCH₂), 71.7 (C-3a), 69.2 and 66.4 (C-2' and C-5'); 65.5 (C-8a), 58.8 (C-4), 46.0 (C-1).

### EXAMPLE 10

### [1R-(1α, 3aβ, 4β, 4aβ, 7β, 7aα, 8aβ)] 8a-[(2,6-Dideoxy-3,4-O-carbonyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 10 (125mg) in refluxing dry toluene (15ml) was added, under nitrogen atmosphere, excess carbonyldiimidazole in small portions. Once the reaction was completed (TLC control), the solvent was removed under reduced pressure and the crude mixture dissolved in dichloromethane (200ml), washed with 1N hydrochloric acid (3x100ml), brine (1x100ml) and water (1x100ml), then dried over magnesium sulfate and concentrated to dryness to give an oil which was used without further purification. The oil was dissolved in ethyl acetate (15ml) and shaken in a Parr apparatus under 20 psi of hydrogen in the presence of 10% palladium on activated charcoal (50mg) for 1 hour at room temperature. Filtration of the catalyst and elimination of the solvent gave a syrup which was purified by preparative TLC (silica gel, methanol:dichloromethane 1:15) to give the title compound (50mg) obtained from the fraction at Rf 0.5.
δ (¹H, CDCl₃): 9.70 (s, 1H, CHO), 6.07 (dd, 1H, H-2, J=1.2 and 3.3 Hz), 4.93 (m, 1H, H-3'), 4.63 (dd, 1H, H-1', J=2.4 and 7.8 Hz), 4.24 (dd, 1H, H-4', J=6.6 and 9.3 Hz), 4.05 and 3.57 (2d, 2H, 8aCH₂, J_{AB}=9 Hz), 3.63 (m, 1H, H-5'), 2.66 (t, 1H, H-1, J=3.9 Hz); δ (¹³C, CDCl₃): 204.6 (COH), 176.0 (COOH), 154.0 (OCO), 148.2 (C-3), 130.7 (C-2), 97.5 (C-1'), 76.6 (C-4'), 74.7 (C-3') 69.9 (C-5'), 31.7 (C-2').

### EXAMPLE 11

### [1R-(1α, 3aβ, 4β, 4aβ, 7β, 7aα, 8aβ)] 8a-[(2,6-Dideoxy-3,4-O-thiocarbonyl-β-D-allopyranosyloxy)methyl]-4-formyl-4,4a,5,6,7,7a,8,8a-octahydro-7-methyl-3-(1-methylethyl)1,4-methano-s-indacene-3a(1H)-carboxylic acid

To a solution of Intermediate 11 (130mg) in dry dichloromethane (5ml) was added trifluoroacetic acid (250µl) at 0^{o}C. After 2 hours the solution was diluted with dichloromethane (50ml), washed with water (2x100ml), dried over magnesium sulfate and concentrated to dryness to give an oil which was purified by preparative TLC (silica gel, methanol:dichloromethane 1:15) to give the title compound (68mg) obtained from the fraction at Rf 0.6.
δ (¹H, CDCl₃): 9.70 (s, 1H, CHO), 6.07 (dd, 1H, H-2, J=1.2 and 3.3 Hz), 5.08 (m, 1H, H-3'), 4.63 (dd, 1H, H-1', J=2.7 and 8.4 Hz), 4.45 (dd, 1H, H-4', J=6.6 and 9.0 Hz), 4.03 and 3.59 (2d, 2H, 8aCH₂, J_{AB}=9.3 Hz), 3.64 (m, 1H, H-5'), 2.67 (t, 1H, H-1, J=3.0 Hz); δ (¹³C, CDCl₃): 204.5 (COH), 190.9 (CS), 176.3 (COOH), 148.2 (C-3), 130.7 (C-2), 97.5 (C-1), 80.3 (C-4'), 79.6 (C-3'), 69.5 (C-5'), 31.3 (C-2').

### EXAMPLE 12

### [1R-(1α, 3aβ, 4β, 4aβ, 7β, 7aα, 8aβ)] 8a-[(2,6-Dideoxy-3,4-O-methylene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 10 (450mg) in dibromomethane (10ml) were added, with vigorous stirring, tetrabutylammonium bromide (40mg) and 50% aqueous sodium hydroxide (100ml). After 12 hours, the mixture was quenched by adding of 1N hydrochloric acid (100ml) and extracted with dichloromethane (100ml). The organic layer was washed with 1N hydrochloric acid (1x100ml), dried over magnesium sulfate and concentrated to dryness to give a white solid which was used without further purification. This solid was dissolved in ethyl acetate (15ml) and shaken in a Parr apparatus under 20 psi of hydrogen in the presence of 10% palladium on activated charcoal (50mg) for 1 hour at room temperature. After filtration of the catalyst and elimination of the solvent, the oily residue was flash chromatographed over silica gel eluting with acetone:hexane (1:10) and (1:5) to give the title compound (130mg) as an oil.
δ (¹H, CDCl₃): 9.80 (s, 1H, CHO), 6.05 (dd, 1H, H-2, J=1.2 and 3.3 Hz), 5.12 and 4.86 (2m, 2H, OCH₂O), 4.60 (dd, 1H, H-1', J=2.4 and 8.7 Hz), 4.20 and 3.44 (2d, 2H, 8aCH₂, J_{AB}=9.3 Hz), 4.13 (m, 1H, H-3'), 3.67 (dd, 1H, H-4', J=5.1 and 8.7 Hz), 3.38 (m, 1H, H-5'), 2.58 (t, 1H, H-1, J=3.9 Hz); δ (¹³C, CDCl₃): 204.6 (COH), 174.8 (COOH), 148.3 (C-3), 130.6 (C-2), 98.1 (C-1'), 94.9 (OCH₂O), 75.4 (C-4'), 73.9 (C-3'), 70.3 (C-5'), 32.3 (C-2').

### EXAMPLE 13

### [1R-(1α, 3aβ, 4β, 4aβ, 7β, 7aα, 8aβ)] 8a-[(2,6-Dideoxy-3,4-O-methylene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid, sodium salt

To a solution of Example 12 (200mg) in methanol (5ml) was added dropwise a 0.106N solution of sodium hydroxide in water (3.98ml). After 30 minutes the solvent was removed under reduced pressure and the residue dissolved in dioxane (5ml) and lyophilized to give the title compound (209mg).
δ (¹H, CD₃OD): 9.87 (s, 1H, CHO), 6.54 (dd, 1H, H-2, J=1.5 and 3.6 Hz), 5.10 and 4.82 (2m, 2H, OCH₂O), 4.51 (dd, 1H, H-1', J=2.4 and 8.7 Hz), 4.12 (m, 1H, H-3'), 4.03 and 3.73 (2d, 2H, 8aCH₂, J_{AB}=9.9 Hz), 3.61 (m, 1H, H-4'), 3.35 (m. 1H, H-5'), 2.56 (t, 1H, H-1); δ (¹³C, CD₃OD): 200.2 (COH), 169.0 (COOH), 142.9 (C-2), 120.6 (C-2), 90.3 (C-1'), 86.4 (OCH₂O), 67.5 (C-4'), 66.2 (C-3'), 61.8 (C-5').

### EXAMPLE 14

### (a) [1R-(1α, 3aβ, 4β, 4aβ, 7β, 7aα, 8aβ)] 8a-[[1S,3R,7R,9R]-2,5,8-Trioxa-3,7-dimethyl-cis-bicyclo[4.4.0]-dec-9-yl-oxy-methyl]-4-formyl-4,4a,5,6,7,7a,8,8a-octahydro-7-methyl-3-(1-methylethyl)-1,4-methano-s-indacene-3a(1H)-carboxylic acid and

### (b) [1R-(1a, 3aβ, 4β, 4aβ, 7β, 7aα, 8aβ)] 8a-[[1S,3S,7R,9R]-2,5,8-Trioxa-3,7-dimethyl-cis-bicyclo[4.4.0]-dec-9-yl-oxy-methyl]-4-formyl-4,4a,5,6,7,7a,8,8a-octahydro-7-methyl-3-(1-methylethyl)-1,4-methano-s-indacene-3a(1H)-carboxylic acid

Intermediate 14 (120mg) in ethyl acetate (15ml) was shaken in a Parr apparatus under 20 psi of hydrogen in the presence of 10% palladium on charcoal (50mg) for 1 hour at room temperature. Filtration of the catalyst and evaporation of the solvent under reduced pressure gave a syrup which was flash chromatographed over silica gel using as eluent acetone:hexane (1:10), (1:7) and (1:5) to give title compound (a) (35mg; Rf=0.5 in hexane:acetone 4:1) and title compound (b) (40mg; Rf=0.4 in hexane:acetone 4:1) both as colourless oils.
(a) δ (¹H, CDCl₃): 9.80 (s, 1H, CHO), 6.04 (dd, 1H, H-2, J=1.2 and 3.3 Hz), 4.62 (dd, 1H, H-9', J=2.1 and 9.9 Hz), 4.20 (m, 2H, 8aCH₂+H-7'), 3.97 (m, 1H, H-1'), 3.73 (m, 1H OCHCH₃), 3.40 (m, 2H, 8aCH₂+OCH₂), 3.27 (d, 1H, OCH₂, J_{AB}=10.8 Hz), 3.18 (dd, 1H, H-6', J=3.3 and 10.2 Hz); δ (¹³C, CDCl₃): 204.6 (COH), 174.6 (COOH), 148.3 (C-3), 130.6 (C-2), 97.9 (C-9'), 73.5 (OCH₂), 73.2 (C-6'), 72.1 (C-1'), 71.9 (OCHCH₃), 65.4 (C-7'), 31.9 (C-10'), 22.7 (OCHCH₃).
(b) δ (¹H, CDCl₃): 9.79 (s, 1H, CHO), 6.05 (dd, 1H, H-2, J=1.5 and 3.6 Hz), 4.73 (dd, 1H, H-9', J=3.0 and 6.3 Hz), 4.26 (m, 1H, H-1'), 4.15 (m, 2H, 8aCH₂+H-7'), 3.98 (m, 1H, OCHCH₃), 3.79 (dd, 1H, OCH₂ , J=3.1 and 11.4 Hz), 3.41 (d, 1H, 8aCH₂, J=9.6 Hz), 3.35 (m, 1H, OCH₂ , J=3.1 and 6.3 Hz), 3.27 (dd, 1H, H-6', J=5.7 and 11.7 Hz), 2.55 (t, 1H, H-1); δ (¹³C, CDCl₃): 204.6 (COH), 174.6 (COOH), 148.3 (C-3), 130.6 (C-2), 99.2 (C-9'), 74.7 (OCHCH₃), 73.6 (OCH₂), 69.3 (C-6'), 66.3 (C-1'), 65.4 (C-7'), 31.9 (C-10'), 22.6 (OCHCH₃).

### EXAMPLE 15

### [1R-(1α, 3aβ, 4β, 4aβ, 7β, 7aα, 8aβ)] 8a-[[1S,7R,9R]-2,5,8-Trioxa-3,3,7-trimethyl-cis-bicyclo[4.4.0]-dec-9-yl-oxy-methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 15 (200mg) in ethyl acetate (15ml) was added 10% palladium on charcoal (50mg) under nitrogen. The mixture was shaken in a Parr apparatus under 20 psi of hydrogen for 1 hour at room temperature. Filtration of the catalyst and evaporation of the solvent gave a residue which was flash chromatographed over silica gel eluting with acetone:hexane (1:10) and (1:15) to give the title compound (140mg) as a white foam.
δ (¹H, CDCl₃): 9.81 (s, 1H, CHO), 6.03 (dd, 1H, H-2, J=1.5 Hz), 4.62 (dd, 1H, H-1', J=2.1 Hz, J=9.6 Hz), 4.35-4.15 (m, 3H, 8aCH₂, H-7' and H-1'), 3.40 (m, 2H, 8aCH₂ and OCH₂), 3.25-3.15 (m, 2H, H-6' and OCH₂), 2.52 (t, 1H, H-1), 1.33 and 1.13 (2s, 2CH₃); δ (¹³C, CDCl₃): 204.7 (COH), 174.1 (COOH), 148.3 (C-3), 130.6 (C-2), 98.0 (C-9'), 73.4 (OCH₂), 73.3 (C-6'), 65.1 (C-1'), 64.7 (C-7), 31.9 (C-10'), 21.1 (2CH₃).

### EXAMPLE 16

### [1R-(1α, 3aβ, 4β, 4aβ, 7β, 7β, 8aβ)] 8a-[[1S,4S,6R,8R]-2,7-Dioxa-4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 20(a) (680mg) in ethyl acetate (100ml) was added 10% palladium on charcoal (200mg) under nitrogen. The mixture was shaken in a Parr apparatus under 25 psi of hydrogen for 2 hours at room temperature. The catalyst was filtered off and the solvent evaporated to dryness. The residue was purified by flash chromatography eluting with dichloromethane:methanol (49:1) to afford the title compound (450mg) as a white foam.
δ(¹H, CDCl₃): 9.86 (s, 1H, CHO), 6.04 (dd, 1H, H-2, J=1.5 and 3.6 Hz), 4.57 (dd, 1H, H-8', J=2.4 and 9.3 Hz), 4.40 (d, 1H, 8a-CH₂, J=9.6 Hz), 4.08 (m, 2H, H-1' and CH₂-3' (1H)), 3.27 (m, 3H, H-6', CH₂-3' (1H), 8a-CH₂ (1H)), 2.47 (t, 1H, H-1, J=3.9 Hz), 1.02 (d, 3H, 4'-CH₃, J=6.6 Hz); δ (¹³C, CDCl₃): 204.7 (CHO), 173.8 (COOH), 148.4 (C-3), 130.5 (C-2), 98.5 (C-8'), 75.2 (C-1'), 74.3 (8a-CH₂), 73.3 (C-3'), 71.9 (C-6'), 51.2 (C-5'), 36.0 (C-4'), 32.9 (C-9'), 19.4(4'-CH₃).

### EXAMPLE 17

### [1R-(1α, 3aβ, 4β, 4aβ, 7β, 7aα, 8aβ)] 8a-[[1S,4S,6R,8R]-2,7-Dioxa-4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid, sodium salt

To a solution of Example 16 (450mg) in methanol (100ml) was added dropwise aqueous sodium hydroxide (0.099 N, 9.5ml). The solvent was removed under reduced pressure and the residue was dissolved in water (80ml) and lyophilized to give the title compound (470mg).
δ (¹H, CD₃OD): 9.86 (s, 1H, CHO), 5.94 (dd, 1H, H-2, J=1.5 and 3.6 Hz), 4.43 (dd, 1H, H-8', J=2.1 and 9.3 Hz), 4.13-4.02 (m, 3H, 8a-CH₂ (1H), H-1', CH₂-3' (1H)), 3.74 (d, 1H, 8a-CH₂, J=9.9 Hz), 3.34-3.23 (m, 2H, CH₂-3' (1H), H-6'), 2.56 (t, 1H, H-1, J=3.9 Hz), 2.34 (m, 1H, H-4') 0.99 (d, 3H, 4'-CH₃, J=6.9 Hz); δ (¹³C, CD₃OD): 209.7 (CHO), 178.4 (COO⁻Na⁺), 152.4 (C-3), 130.1 (C-2), 100.1 (C-8'), 77.2 (C-1'), 77.0 (C-3'), 75.3 (8a-CH₂), 73.0 (C-6'), 52.8 (C-5'), 37.2 (C-4'), 34.5 (C-9'), 19.6 (4'-CH₃).

### EXAMPLE 18

### [1R-(1α, 3aβ, 4β, 4aβ, 7β, 7aα, 8aβ)] 8a-[[1S,4R,6R,8R]-2,7-Dioxa,4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 20(b) (180mg) in ethyl acetate (60ml) was added 10% palladium on charcoal (100mg) under nitrogen. The suspension was shaken in a Parr apparatus under 25 psi of hydrogen for two hours at room temperature. The catalyst was filtered off and the solvent evaporated to dryness. The residue was twice flash chromatographed on silica gel, using successively dichloromethane:methanol (49:1) and hexane:acetone (4:1) as the eluents to give the title compound (90mg) as a white foam.
To a solution of Example 19 (100mg) in methanol (5ml) was added dropwise sodium hydroxide (0.106 N solution in water, 1.88ml). After 30 minutes the solvent was removed under reduced pressure and the residue dissolved in water (3ml) and lyophilized to afford the title compound (104mg).
δ (¹H, CD₃OD): 9.88 (s, 1H, CHO), 5.93 (dd, 1H, H-2, J=1.2 and 3.3 Hz), 4.49 (m, 1H, H-7'), 4.02 (m, 2H, H-3' and 8a CH₂), 3.72 (d, 1H, 8aCH₂, J_{AB}=9.3 Hz), 2.56 (t, 1H, H-1, J=3.6 Hz); δ (¹³C, CD₃OD): 209.7 (CHO), 178.4 (COOH), 152.4 (C-3), 130.1 (C-2), 99.9 (C-7'), 82.1 (C-1'), 76.5 (8aCH₂), 72.2 (C-9'), 37.5 (C-5'), 15.2 (CH₃-CH).

### EXAMPLE 21

### [1R-(1α, 3aβ, 4β, 4aβ, 7β, 7aα, 8aβ)] 8a-[[1S,4S,7R,9R]-2,8-Dioxa-4,9-dimethyl-cis-bicyclo[3.4.0]-non-7-yl-oxy-methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 23(b) (260mg) in ethyl acetate (15ml) was added 10% palladium on charcoal (50mg). The mixture was shaken in a Parr apparatus under 20 psi of hydrogen for 1 hour at room temperature. The catalyst was filtered off and the solvent evaporated to dryness. The residue thus obtained was purified by preparative TLC (silica gel, methanol:dichloromethane 1:20) to afford the title compound (185mg) as an oil at Rf=0.6.
δ (¹H, CDCl₃): 9.85 (s, 1H, CHO), 6.04 (dd, 1H, H-2, J=1.5 and 3.6 Hz), 4.88 (t, 1H, H-7', 2.7 Hz), 4.27 and 3.28 (2d, 2H, 8aCH₂, J_{AB}=9.9 Hz), 3.85 and 3.73 (2m, 2H, OCH₂), 3.60 (m, 1H, H-9'), 3.37 (dd, 1H, H-1', J=7.2 and 8.4 Hz), 2.47 (m, 3H, H-5'+CHCH₃+H1); δ (¹³C, CDCl₃): 204.4 (CHO), 173.7 (COOH), 148.2 (C-3), 130.5 (C-2), 98.7 (C-7'), 81.2 (C-1'), 72.8 (8aCH₂+OCH₂), 69.9 (C-9'), 25.4 (C-6'), 12.4 (CH₃CH).

### EXAMPLE 22

### [1R-(1α, 3aβ, 4β, 4aβ, 7β, 7aα, 8aβ)] 8a-[[1S,7R,9R]-2,8-Dioxa-4,4,9-trimethyl-cis-bicyclo[3.4.0]-non-7-yl-oxy-methyl]-4-formyl-4,4a,5,6,7,7a,8,8a-octahydro-7-methyl-3-(1-methylethyl)-(1,4-methano-s-indacene-3a(1H)-carboxylic acid

To a solution of Intermediate 24 (100mg) in ethyl acetate (40ml) was added 50% palladium on charcoal (50mg) under nitrogen. The mixture was shaken in a Parr apparatus under 25 psi of hydrogen for 2 hours at room temperature. The catalyst was filtered off and the solvent evaporated to dryness. The residue was twice flash chromatographed using successively 1.5% methanol in dichloromethane and 15% acetone in hexane as the eluents to afford the title compound (40mg) as a white foam.
δ (¹H, CDCl₃): 9.85 (s, 1H, CHO), 6.05 (dd, 1H, H-2, J=1.2 and 3.6 Hz), 4.84 (t, 1H, H-7', J=3 Hz), 4.23 and 3.29 (2d, 2H, 8a-CH₂, J=9.6 Hz), 3.81 (t, 1H, H-1', J=8.7 Hz), 3.52 (dq, 1H, H-9', J=9.3 and 6.3 Hz), 3.50 and 3.42 (2d, 2H, CH₂-3', J=8.4 Hz), 2.48 (t, 1H, H-1, J=4.2 Hz), 1.09 and 0.98 (2s, 6H, 4'-CH₃); δ (¹³C, CDCl₃): 204.5 (CHO), 174.0 (COOH), 148.2 (C-3), 130.5 (C-2), 98.7 (C-7'), 81.01 (C-1'), 78.8 (C-3'), 72.7 (8a-CH₂), 70.2 (C-9'), 41.3 (C-4'), 40.4 (C-5'), 27.3 (4'-CH₃), 26.5 (C-6'), 22.8 (4'-CH₃).

### EXAMPLE 23

### [1R-(1α, 3aβ, 4β, 4aβ, 7β, 7aα, 8aβ)] 8a-[(6-Deoxy-3,4-O-isopropylidene-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

To a solution of Intermediate 27 (125mg) in methanol (25ml) was added 10% palladium on charcoal (100mg) under nitrogen. The mixture was shaken in a Parr apparatus under 15 psi of hydrogen for 1.5 hours at room temperature. The catalyst was filtered off and to the filtrate was added 1N hydrochloric acid (0.5ml) at 0^{o}C. After stirring for 1 hour, the mixture was neutralized with 10% sodium hydrogen carbonate. The solvents were evaporated to dryness and the oily residue was purified by flash column chromatography eluting with hexane:ethyl acetate (4:1) and dichloromethane:methanol (20:1) to give the title compound (62mg).
δ (¹H, CDCl₃): 9.73 (s, 1H, CHO), 6.10 (dd, 1H, H-2, J=1.2 and 3.3 Hz), 4.51 (t, 1H, H-2', J=4.5 Hz), 4.48 (d, 1H, H-1', J=7.5 Hz), 3.65 and 4.07 (2d, 2H, 8aCH₂, J=9.3 Hz), 3.78 (dd, 1H, H-4', J=9 and 5.1 Hz), 3.69 (dd, 1H, H-3', J=7.5 and 5.1 Hz), 3.50 (m, 1H, H-5', J=6 and 9 Hz), 2.59 (t, 1H, H-1, J=3.9 Hz), 2.32 (m, 1H, CHMe₂); δ (¹³C, CDCl₃): 204.6 (CHO), 176.1 (CO₂H), 148.2 (C-3), 130.8 (C-2), 110.4 (quaternary C Isopropilidene), 100.9 (C-1'), 78.34 (C-4'), 75.1 (C-2'), 74.7 (8aCH₂), 72.5 (C-4a), 71.7 (C-3'), 69.3 (C-5'), 65.6 (C-8a), 58.9 (C-4), 46.3 (C-1).

### EXAMPLE 24

### [1R-(1α, 3aβ, 4β, 4aβ, 7β, 7aα, 8aβ)] 8a-[(2-Deoxy-3,4-O-isopropylidene-6-O-methyl-β-D-allopyranosyloxy)methyl]-4-formyl-4,4a,5,6,7,7a,8,8a-octahydro-7-methyl-3-(1-methylethyl)-1,4-methano-s-indacene-3a(1H)-carboxylic acid

A solution of Intermediate 30 (50mg) in tetrahydrofuran (5ml) at room temperature was treated with tetrabutylammonium fluoride (1.0 M solution in tetrahydrofuran, 200µl). After 24 hours, the reaction mixture was concentrated to dryness and the resulting yellow oil was purified by flash column chromatography eluting with dichloromethane:methanol (15:1) to give the title compound (18mg).
δ (¹H, CDCl₃): 9.80 (s, 1H, CHO), 5.98 (dd, 1H, H-2, J=1.2 and 3.3 Hz), 4.64 (dd, 1H, H-1', J=2.7 and 8.1 Hz), 4.42 (m, 1H, H-3'), 3.68 and 4.01 (2d, 2H, 8aCH₂, J=9 Hz), 3.90 (m, 1H, H-4'), 3.45-3.65 (m, 3H, H-5' and 2H-6'), 3.22 (s, with 2 % (w/v) yeast extract for 48 hours at 28°C. The organism grows well at temperatures up to 37°C, but not at 45°C. Metachromatic granules were not observed and the strain is catalase positive, oxidase negative and does not metabolise glucose fermentatively. The strain can utilise the following sources of carbon: α-D-glucose, D-fructose, p-hydroxyphenyl-acetic acid, D-mannitol, methylpyruvate, lactamide, D-trehalose and sucrose. The organism can only weakly utilise D-gluconic acid, pyruvic acid and salicin as sole carbon sources. Colony and microscopic morphology resembles that of coryneform bacteria. The genus *Corynebacterium* was excluded on the grounds that the peptidoglycan of NCIMB 40675 contains ornithine rather than the *meso*-isomer of 2,6-diaminopimelic acid or diaminobutyric acid. Also, the organism contains a complex mixture of branch chain fatty acids atypical of *Corynebacterium* species, namely, 12-methyltetradecanoic, 14-methylhexadecanoic and 14-methylpentadecanoic acids. The presence of α-branched-β-hydroxylated fatty acids was not determined. On the basis of these results NCIMB 40675 most closely resembles one of the following actinobacterial genera: *Aureobacterium*, *Curtobacterium* or *Cellulomonas*.
To clarify the taxonomic position of NCIMB 40675, a 1100 base pair partial sequence of the 16S rRNA gene was compared against 24 other species representing a range of actinobacteria and related genera. Results from this analysis indicate a close relationship between NCIMB 40675 and the genera *Aureobacterium* and *Curtobacterium*, but not *Cellulomonas* or *Corynebacterium*. More precise identification for the strain could be obtained by performing a phylogenetic analysis comparing variable regions of the 16S rRNA gene for a range of *Aureobacterium* and *Curtobacterium* species in addition to further chemotaxonomic and physiological tests.

## Claims

1. A compound of formula (I) 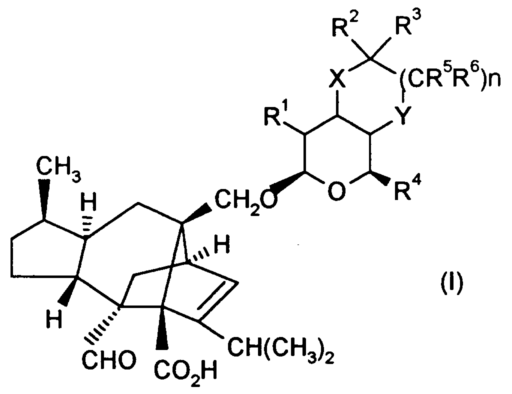 and pharmaceutically acceptable salts and solvates (e.g. hydrates) thereof, wherein R¹ represents hydrogen, halogen, hydroxyl, C₁₋₄alkoxy or acyloxy;
R² and R³ may each independently represent hydrogen, C₁₋₆alkyl or C₁₋₄alkoxyC₁₋₄alkyl or R² and R³ may together with the carbon atom to which they are attached represent C=O, C=S or C₃₋₈cycloalkyl;
R⁴ represents hydrogen or CH₂R⁷ (where R⁷ is hydrogen, hydroxyl, C₁₋₄alkoxy or a group OCOR⁸ in which R⁸ is C₁₋₄alkyl or aryl);
R⁵ and R⁶ may each independently represent hydrogen, C₁₋₆alkyl or C₁₋₄alkoxyC₁₋₄alkyl or R⁵ and R⁶ may together with the carbon atom to which they are attached represent C=O, C=S or C₃₋₈cycloalkyl;
n represents zero or 1;
X and Y may each independently represent oxygen, sulphur or CR⁹R¹⁰ (where R⁹ and R¹⁰ may each independently represent hydrogen, C₁₋₆alkyl or C₁₋₄alkoxyC₁₋₄alkyl or R⁹ and R¹⁰ may together with the carbon atom to which they are attached represent C=O, C=S, C₃₋₈cycloalkyl or C=CHR¹¹ where R¹¹ represents hydrogen or C₁₋₄alkyl); or when X or Y is oxygen and n is zero then -Y-CR²R³ or -X―CR²R³- respectively may also represent -N=CR³- or -NR¹²-CR²R³- (where R² and R³ are as defined previously and R¹² is C₁₋₄alkyl or an acyl group COR¹³ where R¹³ is C₁₋₆alkyl); and the dotted line in formula (I) indicates the optional presence of an additional bond.

2. A compound of formula (Ia) 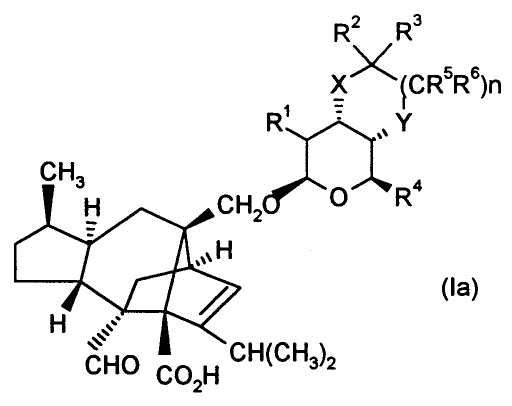 and pharmaceutically acceptable salts and solvates (e.g. hydrates) thereof, wherein R¹ to R⁶, n, X and Y have the meanings defined in Claim 1

3. A compound as claimed in Claim 1 or Claim 2 wherein R¹ is hydrogen

4. A compound as claimed in any one of Claims 1 to 3 wherein R² and R³ each represent individually hydrogen or C₁₋₄alkyl.

5. A compound as claimed in any one of Claims 1 to 4 wherein R⁴ represents methyl.

6. A compound as claimed in any one of Claims 1 to 5 wherein n represents zero.

7. A compound as claimed in any one of Claims 1 to 6 wherein the group 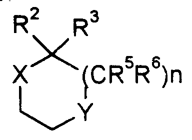 is 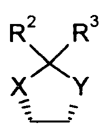 where R² and R³ are as defined previously and X and Y are each independently oxygen, sulphur or CR⁹R¹⁰ provided that at least one of X and Y is oxygen.

8. A compound of the general formula (Ib) 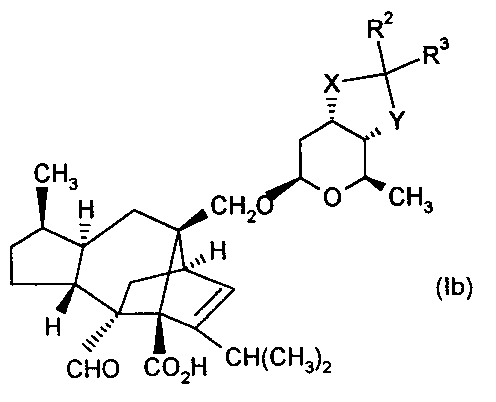 and pharmaceutically acceptable salts and solvates (e.g. hydrates) thereof, wherein R² and R³ are as defined in any of Claims 1 to 7 and one of X and Y represents oxygen and the other represents CR⁹R¹⁰ [where R⁹ and R¹⁰ each independently represent hydrogen or C₁₋₄alkyl or X and Y both represent oxygen.

9. The compounds
[1R-(1α,3aβ,4β,4aβ,7β,7aα,8aβ)] 8a-[(2,6-dideoxy-3,4-*O*-isopropylidene-β-D-allopyranosyloxy)methyl]-4-formyl-4,4a,5,6,7,7a,8,8a-octahydro-7-methyl-3-(1-methylethyl)-1,4-methano-*s*-indacene-3a(1H)-carboxylic acid;
[1R-(1α,3aβ,4β,4aβ,7β,7aα,8aβ)] 8a-[[1S,4S,6R,8R]-2,7-dioxa-4,6-dimethyl-cis-bicyclo[3.4.0]-non-8-yl-oxy-methyl]-4-formyl-4,4a,5,6,7,7a,8,8a-octahydro-7-methyl-3-(1-methylethyl)-1,4-methano-*s*-indacene-3a(1H)-carboxylic acid; and
[1R-(1α,3aβ,4β,4aβ,7β,7aα,8aβ)] 8a-[[1S,4R,7R,9R]-2,8-dioxa-4,9-dimethyl-cis-bicyclo[3.4.0]-non-7-yl-oxy-methyl]-4-formyl-4,4a,5,6,7,7a,8,8a-octahydro-7- methyl-3-(1-methylethyl)-1,4-methano-*s*-indacene-3a(1H)-carboxylic acid; and
pharmaceutically acceptable salts and solvates (e.g. hydrates) thereof.

10. A process for the preparation of a compound of formula (I) which comprises
(a) reacting a compound of formula (II) 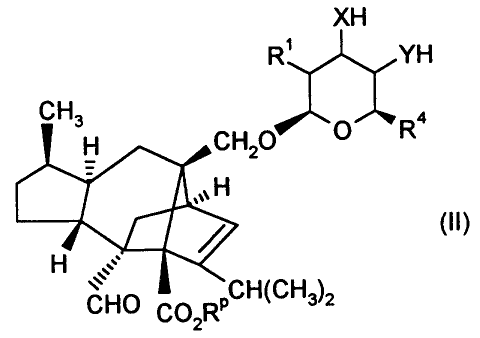 (in which R¹ and R⁴ are as defined in formula (I) above, R^{p} is a carboxyl protecting group and X and Y are as defined in formula (I) above, except that X and/or Y cannot represent CR⁹R¹⁰) to form the desired cyclic system, followed if necessary or desired by one or more of the following steps:
(i) Conversion of one compound of formula (I) are protected derivative thereof into another compound of formula (I)
(ii) Removal of one or more protecting groups

11. A compound as claimed in any one of Claims 1 to 9 for use in therapy.

12. The use of a compound as claimed in any one of Claims 1 to 9 in the manufacture of a medicament for the treatment of fungal infections.

13. A pharmaceutical composition comprising a compound as claimed in any of Claims 1 to 9 in admixture with one or more physiologically acceptable carriers or excipients.

14. A method of treatment of the human or non-human animal body to combat fungal diseases which method comprises administering to said body an effective amount of a compound as claimed in any of Claims 1 to 9.

15. A compound of formula (IX) 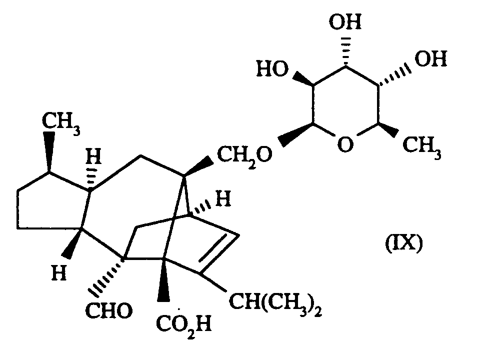 or a protected derivative thereof

16. A process for the preparation of a compound of formula (IX) which comprises
(a) cultivating a microorganism capable of producing the compound of formula (IX) and thereafter isolating the compound of formula (IX) from the culture,
(b) the biotransformation of sordarin.
